# EUROPÄISCHE PATENTSCHRIFT

(11) **EP 3 337 800 B1**
(45) Veröffentlichungstag und Bekanntmachung des Hinweises auf die Patenterteilung: **12.06.2019**
(21) Anmeldenummer: 16753388.4
(22) Anmeldetag: 18.08.2016
(51) Int. Cl.: C07D 471/04, A61K 31/4375, A61P 9/00

(54) **VERFAHREN ZUR HERSTELLUNG VON (4S)- 4-(4-CYANO-2-METHOXYPHENYL)-5-ETHOXY-2,8-DIMETHYL-1,4-DIHYDRO-1,6-NAPHTHYRIDIN-3-CARBOXAMID UND DESSEN AUFREINIGUNG FÜR DIE VERWENDUNG ALS PHARMAZEUTISCHER WIRKSTOFF**
METHOD FOR THE PREPARATION OF (4S)-4-(4-CYANO-2-METHOXYPHENYL)-5-ETHOXY-2,8-DIMETHYL-1,4-DIHYDRO-1-6-NAPHTHYRIDINE-3-CARBOXAMIDE AND THE PURIFICATION THEREOF FOR USE AS AN ACTIVE PHARMACEUTICAL INGREDIENT
PROCÉDÉ DE PRÉPARATION DE (4S)-4-(4-CYANO-2-MÉTHOXYPHÉNYL)-5-ÉTHOXY-2,8-DIMÉTHYL-1,4-DIHYDRO-1,6-NAPHTYRIDINE-3-CARBOXAMIDE ET DE PURIFICATION DE CE DERNIER AFIN DE L'UTILISER EN TANT QUE PRINCIPE ACTIF PHARMACEUTIQUE

(30) Priorität: 21.08.2015 EP 15182043
(43) Veröffentlichungstag der Anmeldung: 27.06.2018
(73) Patentinhaber: Bayer Pharma Aktiengesellschaft, 13353 Berlin (DE)
(72) Erfinder: PLATZEK, Johannes, 12621 Berlin (DE)
(74) Vertreter: BIP Patents
(86) Internationale Anmeldenummer: PCT/EP2016/069558
(87) Internationale Veröffentlichungsnummer: WO 2017/032673

(56) Entgegenhaltungen:
- WO-A1-2016/016287
- WO-A2-2008/104306
- LARS BÄRFACKER ET AL: "Discovery of BAY 94-8862: A Nonsteroidal Antagonist of the Mineralocorticoid Receptor for the Treatment of Cardiorenal Diseases", CHEMMEDCHEM, Bd. 7, Nr. 8, 12. August 2012 (2012-08-12) , Seiten 1385-1403, XP055213619, ISSN: 1860-7179, DOI: 10.1002/cmdc.201200081 in der Anmeldung erwähnt

## Beschreibung

Die vorliegende Erfindung betrifft ein neues und verbessertes Verfahren zur Herstellung von (4S)- 4-(4-Cyano-2-methoxyphenyl)-5-ethoxy-2,8-dimethyl-1,4-dihydro-1,6-naphthyridin-3-carbox-amid der Formel (I)

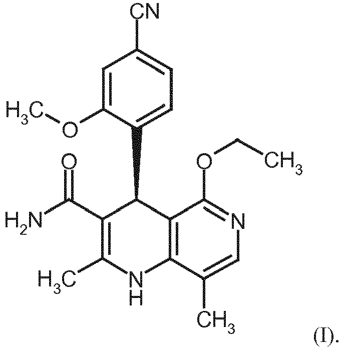

Die Verbindung der Formel (I) wirkt als nicht steroidaler Antagonist des Mineralcorticoid Rezeptors und kann als Mittel zur Prophylaxe und /oder Behandlung von kardiovaskulären und renalen Erkrankungen wie beispielsweise Herzinsuffizienzen und diabetische Nephropathie eingesetzt werden.

Die Verbindung der Formel (I) und deren Herstellungsprozess sind in der WO 2008/104306 und ChemMedChem 2012, 7, 1385 beschrieben, wobei in beiden Publikationen eine ausführliche Diskussion der Forschungs-Synthese offenbart ist. Nachteilig an der dort beschriebenen Synthese ist die Tatsache, dass sich diese Synthese nicht für ein weiteres großtechnisches Verfahren eignet, da viele Schritte in sehr hoher Verdünnung, mit sehr hohen Reagenz-Überschüssen und damit zu einer relativ geringen Gesamtausbeute verlaufen. Des Weiteren sind viele chromatographische Zwischenreinigungen notwendig, die in der Regel technisch sehr aufwendig sind und einen hohen Verbrauch an Lösungsmitteln erfordern, kostenintensiv sind und damit nach Möglichkeit vermieden werden sollten. Einige Stufen lassen sich aufgrund von sicherheits- und verfahrenstechnischen Schwierigkeiten nicht im großtechnischen Verfahren realisieren.

Es bestand daher der Bedarf nach einer großtechnisch praktikablen Synthese, die die Verbindung der Formel (I) reproduzierbar in hoher Gesamtausbeute, niedrigen Gestehungskosten und hoher Reinheit liefert und allen regulatorischen Anforderungen entspricht, um die klinischen Prüfungen mit Wirkstoff zu beliefern und für eine spätere behördliche Einreichung verwendet zu werden.

Mit der vorliegenden Erfindung wurde eine sehr effiziente Synthese gefunden, die es erlaubt die oben genannten Anforderungen zu erfüllen.

In der Publikation ChemMedChem 2012, 7, 1385 in der die Forschungs-Synthese der Verbindung der Formel (I) offenbart ist, wird ausgehend von Vanillin die Verbindung der Formel (I) in 10 Stufen mit einer Gesamtausbeute von 3,76 % der Theorie hergestellt. Die Verbindung der Formel (I) wurde durch Eindampfen von Chromatographie-Fraktionen als amorpher Feststoff erhalten, ein definiertes Kristallisations-Verfahren der Endstufe zur Polymorphie-Einstellung ist bisher nicht beschrieben.

Das nachfolgende Schema 1 zeigt das bekannte Verfahren zur Herstellung der Verbindung der Formel (I).

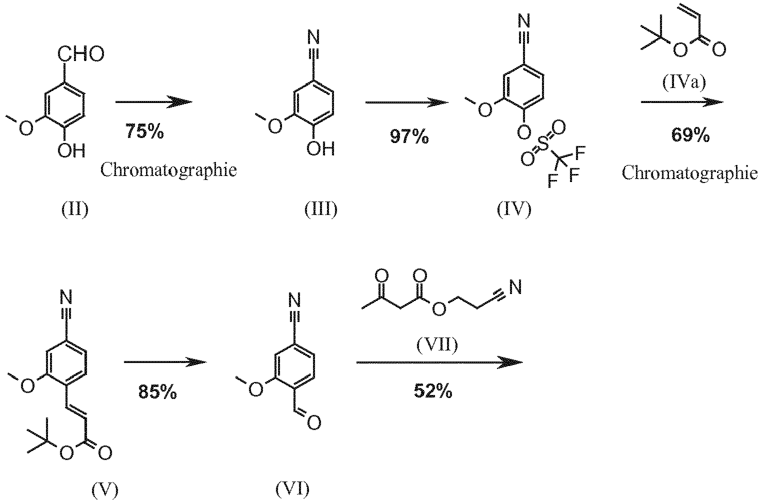

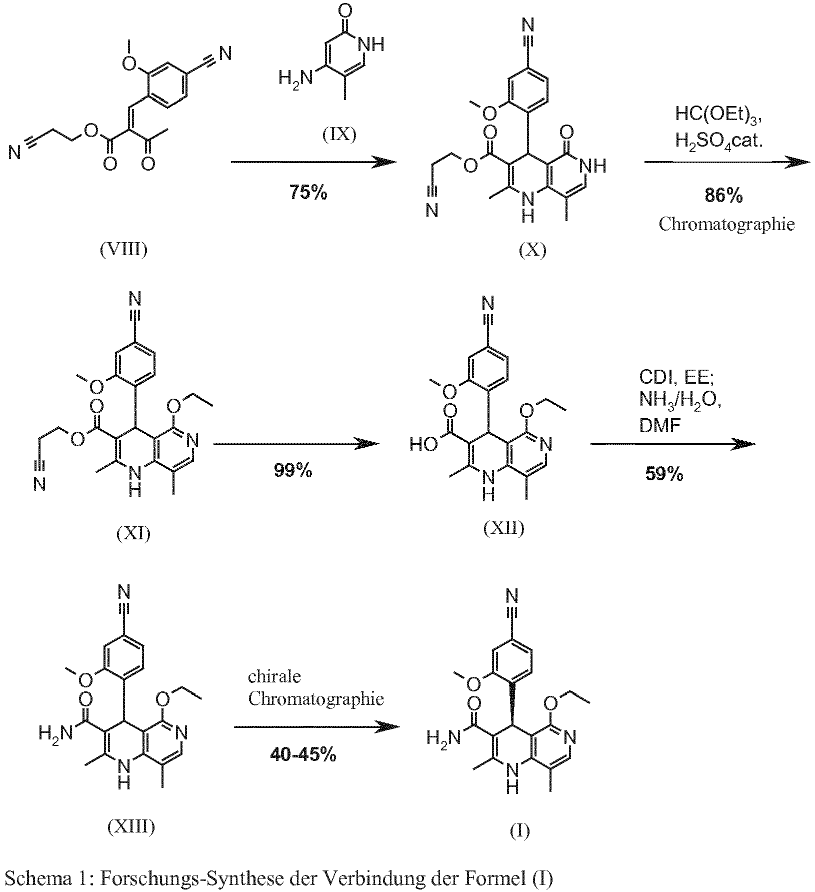

Es werden 3 chromatographische Aufreinigungen benutzt, sowie eine chirale Chromatographie-Stufe zur Auftrennung der Enantiomere des Racemates der Formel (XIII). Die Stufen laufen teilweise in sehr starker Verdünnung und unter Einsatz sehr großer Reagenz-Mengen.

So ist insbesondere auch die Sequenz der Herstellung der Nitril-Aldehyd Zwischenstufe (VI), die eine zentrale Rolle in dieser Synthese einnimmt aus atomökonomischer Sicht nicht akzeptabel.

Schema 2 veranschaulicht das neue erfindungsgemäße Verfahren, ausgehend vom Nitrilaldehyd (VI) welches die Verbindung der Formel (I) in 4 Stufen in 31.5 % d. Th. Gesamtausbeute (8.8 % der obigen Forschungs-Synthese ausgehend vom Aldehyd (VI)) ohne eine chromatographische Aufreinigung von Zwischenstufen liefert.

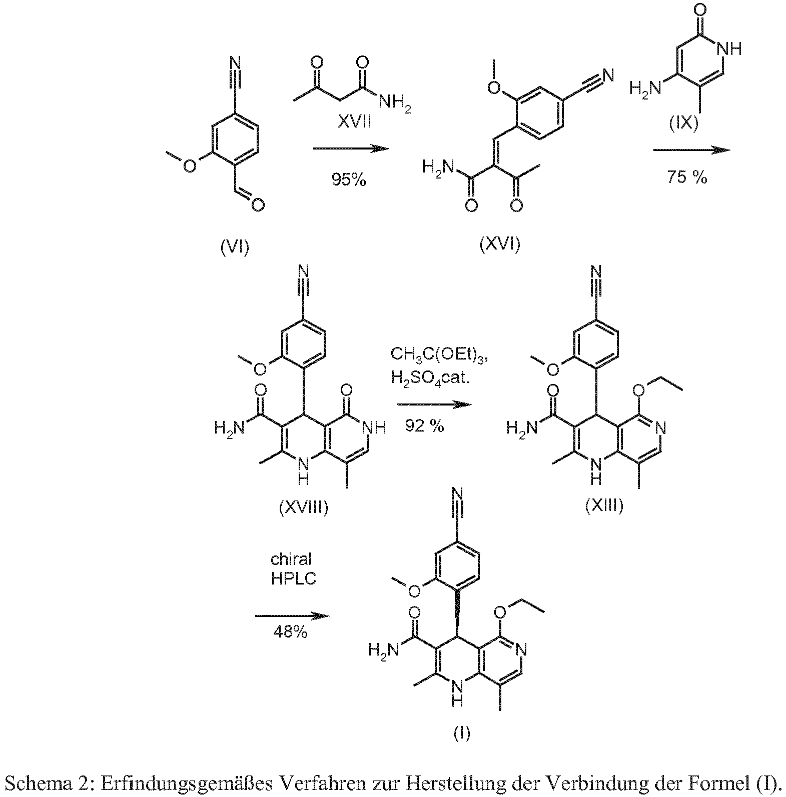

Zur Enantiomeren-Trennung wird eine präparative chirale HPLC-Methode (z.B. SMB-Technologie, Varicol) benutzt.

Der Aldehyd (VI) ist literaturbekannt (J. Med. Chem. 2007, 50, 2468-2485) und stellt eine wichtige Zwischenstufe dieser Synthese dar. Es gibt mittlerweile auch die Möglichkeit die Verbindung käuflich zu erwerben.

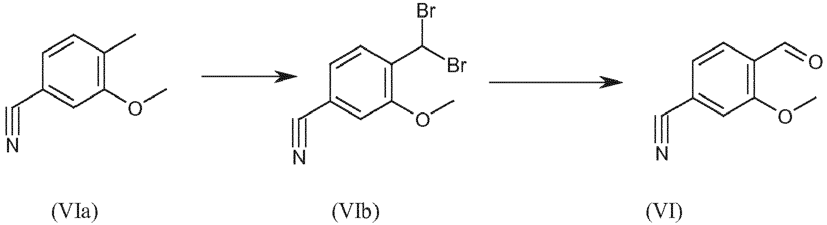

Ausgehend vom 4-Cyano-2-methoxytoluol (VIa) wird mit NBS ein Dibromid (VIb) hergestellt, welches in Ethanol mit 2,46 eq. Silbernitrat (in Wasser) zum Zielaldehyd (VI) umgesetzt wird. Diese in der Literatur beschriebene Synthese sowie das in der Forschungs-Synthese beschriebene Verfahren sind für ein Up-Scaling in den Multi-Tonnen-Maßstab völlig ungeeignet, sodass ein hoher Bedarf nach einer neuen effizienteren und ökonomisch günstigeren Synthese bestand.

Die Halogenbenzoesäuren (XIV) und (XIVa) 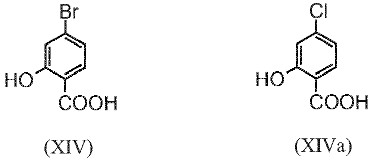 sind in größeren Mengen kommerziell erhältlich. Es wurde ein sehr effizienter und preiswerter Prozess entwickelt, bei dem die Zwischenstufen (XV) und (XIX) 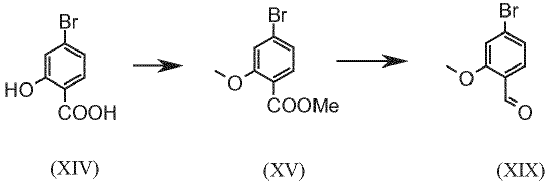 nicht isoliert, sondern in Lösung gelöst, weiter umgesetzt werden. Dieses ist nur dadurch möglich, weil die Ausbeute und Reinheit der jeweiligen Umsetzung sehr hoch ist (> 95%.Th.). Der Methylether-Ester (XV) ist literaturbekannt (Journal of Medicinal Chemistry, 1992, vol. 35, p. 734 - 740) und wird durch Umsetzung mit dem sehr leichtflüchtigem, gesundheitsschädlichen und teuren Methyljodid hergestellt.

Es konnte gezeigt werden, dass das schwerflüchtige, preiswertere Dimethylsulfat analog verwendet werden kann. Ausgehend von der Säure (XIV) wird in einem Lösungsmittel wie Aceton, 2-Butanon, THF, 2-Methyl-THF, DMF, DMA oder NMP mit Dimethylsulfat unter Zuhilfenahme einer Hilfsbase wie Kaliumcarbonat, Natriumcarbonat, Calciumcarbonat, Lithiumcarbonat, N-Methylimidazol, Triethylamin, Pyridin, 2,6-Lutidin bei Temperaturen von 50-100°C zum Methylether-Ester (XV) umgesetzt. Hierbei handelt es sich um eine dem Fachmann bekannte Methode der Veresterung von Säuren und Veretherung von Phenolen (Tetrahedron, 2013, vol. 69, p. 2807 - 2815, Journal of the American Chemical Society, 2013, vol. 135, p. 5656 - 5668). Als besonders bevorzugt hat sich die Umsetzung in Aceton unter Rückfluss (56°C) unter Verwendung von Dimethylsulfat und Kaliumcarbonat herausgestellt. Hierzu wird Dimethylsulfat innerhalb von 4 Stunden zur siedenden Reaktionsmischung zu dosiert. Das Aceton wird abdestilliert und durch Toluol ersetzt (Umdestillation). Zur Aufarbeitung wird Wasser zugesetzt (Zerstörung des überschüssigen Dimethylsulfates), die Toluolphase abgetrennt und mit Wasser und gesättigter Kochsalz-Lösung gewaschen und die Toluol-Lösung anschließend bis auf ein bestimmtes Volumen abdestilliert (dient zur azeotropen Trocknung, d.h. Entfernung von Wasser für die Folgestufe). Eine Gehaltsbestimmung der Lösung zeigt einen nahezu vollständigen Umsatz (> 96 % d. Th.) an. Anstelle der Bromverbindung kann analog die Chlorverbindung eingesetzt werden, die erzielten Umsätze sind identisch mit der Bromverbindung.

Die Herstellung des Aldehyds (XIX) ist in der Literatur beschrieben, beispielhaft genannt seien: Glaxo Group Limited US2008/312209 A1, 2008, European Journal of Medicinal Chemistry, 1986, vol. 21, p. 397 - 402, Journal of Medicinal Chemistry, 1992, vol. 35, p. 734 - 740, Journal of Materials Chemistry, 2011, vol. 21, p. 9523 - 9531. Jedoch sind die in den Umsetzungen eingesetzten Ausgangsstoffe sehr teuer und nicht in großen Mengen erhältlich, daher wurde ausgehend vom Methylether-Ester (XV) ein neues Verfahren entwickelt. Die Umsetzung von (XV) zum Aldehyd (XIX) gelingt mit REDAL (Natrium-bis-(2-methoxy-ethoxy)-aluminium-dihydrid) in Toluol unter Zusatz von N-Methylpiperazin. Diese Methode ist in der Literatur beschrieben (Synthesis 2003, No. 6, 823-828 und Tetrahedron 57 (2001) 2701-2710). Führt man die Reaktion analog der in der Literatur angegebenen Stöchiometrie durch, so wird neben dem Aldehyd noch eine weitere Verbindung im Ansatz gefunden. Es zeigte sich, dass es sich um den korrespondierenden Benzylalkohol handelt, der durch Überreduktion bis zu 10 % entsteht. Es zeigte sich, dass es wichtig ist die Stöchiometrie des REDAL und N-Methylpiperazin exakt einzustellen, 1,21 eq. REDAL + 1,28 eq. N-Methylpiperazin, dann gelingt es, dieses Nebenprodukt, welches in der Folgestufe die Kristallisation stört, bis auf < 1% zu reduzieren. Hierzu wird eine 65%ige REDAL-Lösung in Toluol bei 0-5°C vorgelegt (bevorzugt 1,21 eq.) und 1,28 eq. N-Methylpiperazin zudosiert. Die so erhaltene Lösung von REDAL mit N-Methylpiperazin wird innerhalb ca. 30 Minuten zur vorgelegten Brommethylester-Lösung (XIV) in Toluol zudosiert und anschließend eine Stunde bei 0°C gerührt. Die Reaktionslösung wird auf Wasser/Säure, bevorzugt aqu. Schwefelsäure gequenscht und die Toluol-Phase abgetrennt und mit Wasser und gesättigter Kochsalzlösung gewaschen. Das Toluol wird abdestilliert und auf DMF (Lösungsmittel der Folgestufe) umdestilliert. Die Ausbeute der Umsetzung beträgt in der Regel > 94% d. Theorie. Die entsprechende Umsetzung mit der Chlorverbindung läuft analog, die Ausbeuten sind entsprechend. Die DMF-Lösung wird direkt in die Folgereaktion eingesetzt.

Im weiteren Verlauf der Synthese wird der Bromaldehyd (XIX) in an sich bekannter Weise nach dem Fachmann geläufigen Methoden (Synth. Commun. 1994, 887-890, Angew. Chemie 2003, 1700-1703, Tetrahedron Lett. 2007, 2555-2557, Tetrahedron Lett. 2004, 1441-1444, JACS 2003, 125, 2890-2891, Journal of Organometallic Chemistry 689 (2004), 4576-4583) in das Nitril überführt, hierbei erhält man den Nitrilaldehyd (VI). Im Falle der Bromverbindung hat es sich als besonders vorteilhaft erwiesen, eine Palladium katalysierte Umsetzung mit Kaliumhexacyanoferrat * 3 H₂O als Cyanid-Quelle durchzuführen (Tetrahedron Lett. 48 (2007), 1087-1090). Hierzu wird der Bromaldehyd (XIX) in DMF (8- 10 fach) vorgelegt, 0,22 eq. Kaliumhexacyanoferrat * 3 H₂O und 1 eq. Natriumcarbonat vorgelegt und anschließend 0,005 eq. Palladiumacetat zugesetzt. Man erwärmt 3 Stunden auf 120°C. Die Lösung wird auf 20°C abgekühlt, anschließend Wasser und Essigester zugesetzt. Die Essigester Phase wird abgetrennt, die Wasser Phase erneut mit Essigester nachgewaschen und anschließend die vereinigten Essigester-Phasen auf Isopropanol umdestilliert. Man fällt das Produkt durch Wasser Fällung in der Siedehitze aus. Nach Isolierung wird im Vakuum getrocknet. In manchen Fällen wurde das Produkt direkt durch Zugabe von Wasser aus dem DMF ausgefällt und nach Isolierung und Trocknung direkt in die Folgestufe eingesetzt. Die Ausbeuten dieser Umsetzung sind in der Regel > 85 % d.Th. Für die Umsetzung der Chlorverbindung reicht Palladiumacetat nicht aus, hier hat es sich als vorteilhaft erwiesen die dem Fachmann geläufigen Palladium-Katalysatoren, wie in Tetrahedron Lett. 48 (2007), 1087-1090 beschrieben, einzusetzen, die Ausbeuten sind etwas niedriger als bei der Bromverbindung, in der Regel 80-85 % d.Th.

Das Zimtamid (XVI a,b) erhält man ausgehend vom Aldehyd der Formel (VI) in einer Knoevenagel-Reaktion mit dem Ketoamid (XVII) als E/Z-Gemisch :

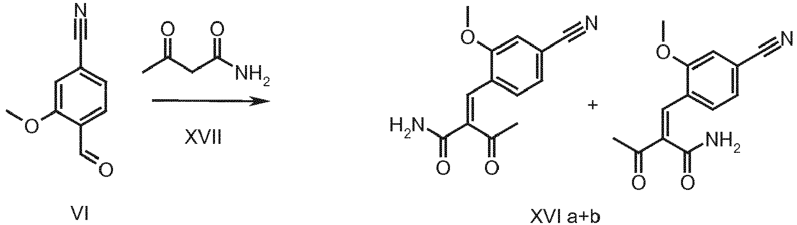

Die Reaktion läuft bevorzugt in siedendem Dichlormethan (10-fach - 20-fach) unter Zusatz von 5-20 mol% Piperidin, bevorzugt 10 mol% und 5-20 mol% Eisessig, bevorzugt 5 - 10 mol% am Wasserabscheider ab. Die Reaktionszeit beträgt 4-12 h, bevorzugt aber 5-6 h, besonders bevorzugt 6 h. Das Ketoamid (XVII) wird in 1,0-1,5 eq, bevorzugt aber 1,1 bis 1,35 eq. zugesetzt. Besonders bevorzugt 1,1 eq. Die Herstellung des Ketoamids (XVII) ist bekannt und in Tetrahedron Letters, 1993, vol. 34, 6141 - 6142 beschrieben, aber auch als Kaufware erhältlich. Nach beendeter Reaktion wird auf 20 °C abgekühlt und die organische Phase 2mal mit Wasser gewaschen. Die organische Wasche wird auf 2-Butanol umdestilliert und das E/Z-Zimtamidgemisch (XVI a+b) ohne Zwischenisolierung direkt die Folgereaktion mit dem Heterocyclus (IX) zum Dihydropydidin (XVIII) eingesetzt:

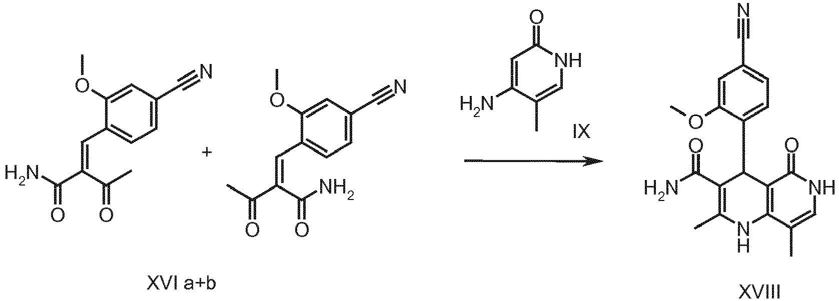

Es wurde gefunden, dass die Reaktion bevorzugt in Alkoholen, wie Ethanol, Isopropanol, Isobutanol (2-Butanol), 2-Amylalkohol, Cyclohexanol bei Temperaturen von 80-160°C, unter Normaldruck, sowie im Autoklaven (2-10 bar), Reaktionszeiten 8-40 h, bevorzugt aber in Isopropanol im Autoklaven (100°C-130°C, 2-10 bar, bevorzugt 3-5 bar, 8-24 h) oder in Ethanol (90-130°C, 3-10 bar, 3-24 h) in 2-Butanol (100°C-130°C, 2-10 bar, bevorzugt 3-5 bar, 8-24 h) durchgeführt wird. Zur Aufarbeitung wird auf 0°C bis 20°C abgekühlt, die Kristalle abfiltriert und mit Ethanol nachgewaschen, anschließend getrocknet (im Vakuum, 60 °C).

Wenn auf die Verwendung von Dichlormethan aus umweltökonomischen Gründen verzichtet werden soll, hat sich als vorteilhaft erwiesen den Zimtamid (XVI a,b) in Isopropanol herzustellen, hierzu wird der Aldehyd (VI) in Isopropanol (3-9 fach, bevorzugt 5-7 fach) vorgelegt und 5-20 mol% Piperidin, bevorzugt 5 - 10 mol% und 5-20 mol% Eisessig, bevorzugt 5 - 10 mol% zugegeben. Bei 30°C wird 1,0-1,5 eq, bevorzugt 1,1 - 1,35 eq., besonders bevorzugt 1,1 eq. Ketoamid (XVII), gegebenenfalls in wenig Isopropanol gelöst, über 3 Stunden zudosiert und 1 Stunde bei 30°C nachgerührt. Der Zimtamid (XVI a,b) kristallisiert während der Reaktion aus. Anschließend wird das Produkt, gegebenenfalls nach Abkühlen, bevorzugt bei 0°C abfiltriert, mit wenig Isopropanol (auf 0°C gekühlt) gewaschen und feucht in die Folgereaktion wie oben beschrieben eingesetzt. Die Ausbeute beträgt > 95 % der Theorie. Bevorzugt wird in der Folgeumsetzung in 10- 15 fach (bzg. auf Aldehyd (VI)), bevorzugt 10-12 fach 2-Butanol oder Isopropanol 20-24 Stunden bei 100°C unter Druck gearbeitet. Das Produkt wird nach beendeter Reaktion und Abkühlen durch Filtration oder Zentrifugation isoliert. Anschließend wir bei 40 - 90°C im Vakuum getrocknet. Da der Umsatz zum Zimtamid (XVI a,b) nahezu quantitativ verläuft, kann man den Prozess für die Folgestufe leicht standardisieren, ohne jeweils die Menge an Heterocylus (IX) anpassen zu müssen, daher kann das Produkt Isopropanol feucht eingesetzt werden. Die Ausbeuten liegen bei > 75 % d. Theorie. Der Heterocyclus (IX) kann nach literaturbekannten Methoden, wie z.B. in Synthesis 1984, 765-766 beschrieben, hergestellt werden.

Ausgehend vom Dihydropyridin (XVIII) wird der Ethylether (XIII) durch Umsetzung unter saurerer Katalyse mit einem Orthoester (XX) erhalten, wobei R für -H und -Methyl steht:

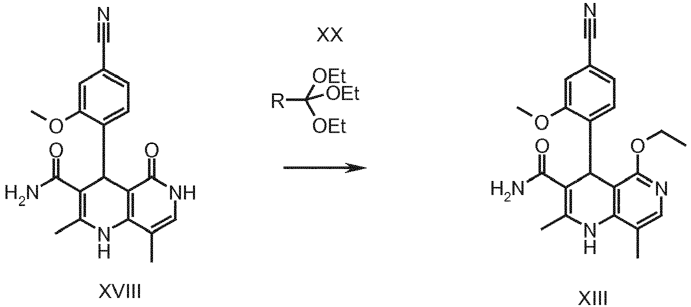

Es wurde überraschenderweise gefunden, dass die Reaktion sehr konzentriert (bis zu 1,5 g Lösungsmittel auf 1 g Edukt) in Lösungsmitteln wie Dimethylacetamid, NMP (1-Methyl-2-pyrrolidon), DMF (Dimethylformamid) unter Zusatz von 4-10 gew. % konz. Schwefelsäure, bevorzugt 6-8 gew. % durchgeführt werden kann. Die Reaktion läuft dann überraschenderweise bereits mit 2,5 - 5 equ. Orthoester (XX), (R= H oder Me). Es wurde gefunden, dass es viel günstiger ist, den entsprechenden Triethylorthoessigester in die Reaktion einzusetzen, da er zum einen viel sauberer reagiert und viel schwerer entflammbar ist, was ihn für die technische Durchführung besonders prädestiniert. Die Umsetzung erfolgt bevorzugt in DMA (Dimethylacetamid) und NMP (1-Methyl-2-pyrrolidon), bei Temperaturen von 100-120°C, bevorzugt 115 °C. Es hat sich als vorteilhaft erwiesen, vor Start der eigentlichen Reaktion ein Teil des Lösungsmittels (DMD oder NMP) bei erhöhter Temperatur (100 -120°C unter Vakuum) abzudestillieren, um gegebenenfalls vorhandene Reste an Isopropanol aus der Vorstufe zu entfernen, da ansonsten unerwünschte Nebenprodukte auftreten. Umsetzung: Man rührt 1,5 - 3 Stunden, bevorzugt 2 Stunden. Zur Aufarbeitung wird Wasser direkt in den Ansatz gegeben, wobei das Produkt auskristallisiert. Um ein besonders stabiles und reproduzierbares Verfahren in der Hand zu haben, wir erst eine Teilmenge Wasser (z.B. 1/3) zudosiert, dann angeimpft und die Restmenge Wasser zugegeben. Dieses Vorgehen garantiert, dass man immer die gleiche Kristallmodifikation erhält, die das beste Isolierverhalten zeigt. Das Produkt wird mit Wasser nachgewaschen und getrocknet. Die Ausbeuten liegen > 92 % d. Theorie. Die Reinheit ist in der Regel > 99% (HPLC, 100%-Methode).

Um an die Verbindung der Formel (I) zu gelangen, muss das racemische Gemisch der Amide (XIII) in die Antipoden getrennt werden. In der publizierten Forschungs-Synthese wurde hierzu eine speziell synthetisierte chirale Phase verwendet (Eigenherstellung), die als chiralen Selektor N-(dicyclopropylmethyl)-N²-methacryloyl-D-leucinamid enthielt. Dieser Selektor wurde in einer Mehrstufen-Stufe hergestellt und dann auf spezielles Kieselgel aufpolymerisiert. Als Laufmittel diente Methanol/Essigester. Ein großer Nachteil dieser Methode war die sehr geringe Beladung, 30 mg pro Trennung auf einer 500*63 mm Chromatographie-Säule, sodass ein hoher Bedarf bestand eine möglichst effektive Trennmethode zu finden, die es erlaubt eine Antipoden-Trennung im multi Tonnen-Bereich vorzunehmen. Es wurde überraschenderweise gefunden, dass man die Trennung auch auf einer kommerziell leicht zugänglichen Phase vornehmen kann. Hierbei handelt es sich um die Phase Chiralpak AS-V, 20 µm. Als Laufmittel wurde eine Mischung aus Methanol/Acetonitril 60:40 verwendet. Diese Mischung hat den großen Vorteil, dass es nach destillativer Aufarbeitung als Laufmittel wieder zurückgewonnen werden kann, mit der identischen Zusammensetzung (60:40 entspricht dem Azeotrop). Auf diese Weise erreicht man einen sehr effizienten Prozess, bei der die Ausbeute der Trennung > 47 % d. Theorie beträgt (50% sind theoretisch möglich). Die optische Reinheit beträgt hier > 93 % e.e. bevorzugt aber > 98,5 % e.e. Hierbei kann die Chromatographie an einer handelsüblichen Chromatographie-Säule durchgeführt werden, bevorzugt werden aber mit dem Fachmann bekannten Techniken wie SMB oder Varicol (Computers and Chemical Engineering 27 (2003) 1883-1901) eingesetzt. So wurde beispielsweise mit einer SMB Anlage ca. 500 kg des racemischen Amids (XIII) getrennt, wobei eine Ausbeute von 48% erzielt wurde. Das Produkt bringt man als 3 - 8 %ige, bevorzugt 5 -7 %ige Lösung in einem Gemisch aus Methanol/Acetonitril 60:40 aus und kann es direkt ins "Final Processing" einsetzen. Auch andere Lösungsmittelgemisch-Verhältnisse zwischen Acetonitril und Methanol sind denkbar (90:10 bis 10:90). Es können aber alternativ auch andere Lösungsmittel-Gemische, wie Acetonitril/Ethanol in Gemisch-Verhältnissen von 10:90 bis 90:10 zur SMB Trennung verwendet werden. Das jeweilige Verhältnis der Lösungsmittel hängt teilweise von den technischen Eigenschaften der SMB-Anlage ab und muss gegebenenfalls angepasst werden (z.B. verschiedene Flussrate, Recycling der Lösungsmittel am Dünnschicht-Verdampfer).

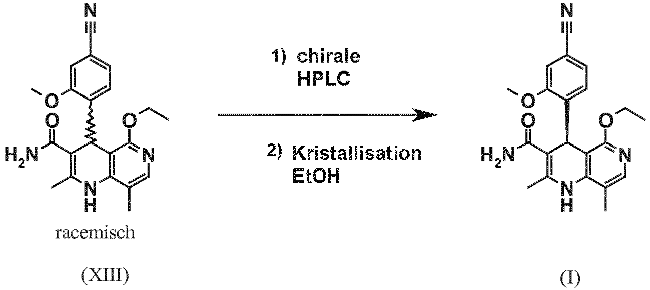

Da die Verbindung der Formel (I) in Form einer Tablette entwickelt wird, besteht ein hoher Bedarf dafür, dass man die isolierte Verbindung der Formel (I) reproduzierbar in einer definierten kristallinen Form isoliert, sodass man eine reproduzierbare Bioverfügbarkeit gewährleisten kann. Es wurde überaschenderweise gefunden, dass man die Verbindung der Formel (I) aus Methanol, Ethanol, THF, Acetonitril, sowie deren Mischungen mit Wasser kristallisieren kann, wobei reproduzierbar nur ein Polymorph I entsteht, das einen definierten Schmelzpunkt von 252 °C besitzt. Vorteilhafter Weise wird Ethanol, bzw. vergälltes Ethanol verwendet.

Finales Kristallisations-Verfahren: Hierzu wird aus GMP-technischen Gründen die aus der Chromatographie kommende ca.5-7% ige Produkt-Lösung in Methanol/Acetonitril 60:40 (oder, falls in Ethanol/Acetonitril gearbeitet wurde, eine ca. 3-4 % ige Lösung aus Ethanol/Acetonitril 50:50) zuerst einer Partikelfiltration unterzogen und anschließend ein Lösungsmittel-Tausch auf Ethanol vorgenommen, bevorzugt wird mit Toluol vergälltes Ethanol eingesetzt. Hierzu wird mehrmals umdestilliert, eingeengt und jeweils frisches Ethanol zugesetzt. Nach Austausch wird so viel Ethanol zugegeben bis in der Siedehitze eine Lösungsphase durchlaufen wird und dann unter Normaldruck, bzw. unter leicht reduziertem Druck auf ca. 3 bis 4fach Volumen eingeengt, dabei kristallisiert das Produkt aus. Es wird auf 0°C abgekühlt und anschließend die Kristalle isoliert und im Vakuum bei 40 - 50 °C getrocknet. Die Ausbeuten sind in der Regel > 90% d. Theorie. Die erzielte chemische, Reinheit ist > 99,8 % und der Gehalt ∼ 100 % entsprechen den Kriterien für Handelsprodukte nach ICH-Guideline. Restlösungsmittel, in dem Fall Ethanol, ist < 0,02 %. Die optische Reinheit beträgt >> 99 % e.e.

Die Verbindung der Formel (I) liegt in kristalliner Form der Modifikation I vor 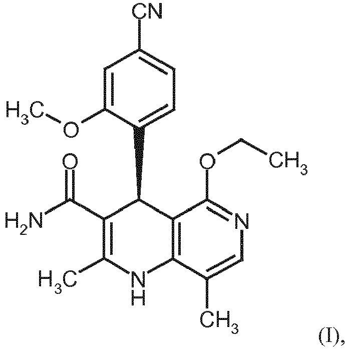 dadurch gekennzeichnet, dass das Röntgendiffraktogramm der Verbindung Peakmaxima des 2 Theta Winkels bei 8.5, 14.1, 17.2, 19.0, 20.5, 25.6, 26.5 zeigt.

Die Verbindung der Formel (I) liegt in kristalliner Form der Modifikation I vor 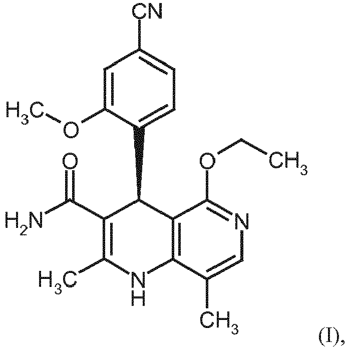 dadurch gekennzeichnet, dass das IR-Spektrum (IR-ATR) der Verbindung Bandenmaxima bei 3475, 2230, 1681, 1658, 1606, 1572, 1485, 1255, 1136 und 1031 cm⁻¹ zeigt.

Die Verbindung der Formel (I) liegt in kristalliner Form der Modifikation I vor 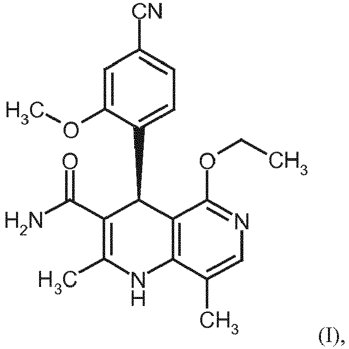 dadurch gekennzeichnet, dass das Raman-Spektrum der Verbindung Bandenmaxima bei 3074, 2920, 2231, 1601, 1577, 1443, 1327, 1267, 827 und 155 cm⁻¹ zeigt.

Die Verbindung der Formel (I) wird in der Regel mikronisiert und in der Pharmazie zu Tabletten formuliert. Es zeigt sich, daß die Verbindung der Formel (I) in kristalliner Form der Modifikation I sehr gute Stabilitäts-Eigenschaften (auch bei hoher Luftfeuchtigkeit) besitzt und über > 2 Jahre hinweg problemlos gelagert werden kann.

Mit der erfindungsgemäßen Synthese ist es gelungen in sehr effizienter Weise die Verbindung der Formel (I) herzustellen. Das Verfahren bietet gegenüber dem Stand der Technik erhebliche Vorteile, was die Skalierbarkeit und technische Durchführung betrifft. Die Gesamtausbeute ist verglichen mit publizierten Daten signifikant höher, außerdem werden exzellente Reinheiten beim Wirkstoff erzielt. Das neue Verfahren ermöglicht die reproduzierbare, ökonomische Herstellung der definierten Verbindung der Formel (I) in kristalliner Form der Modifikation I, dessen Existenz im Stand der Technik noch nirgendwo beschrieben wurde.

Gegenstand der vorliegenden Erfindung ist ein Verfahren zur Herstellung der Verbindung der Formel (I) 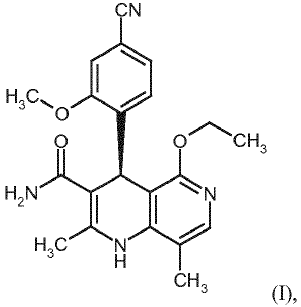 dadurch gekennzeichnet, dass die racemische Verbindung der Formel (XIII) in ihre Enantiomere getrennt wird, und die Verbindung der Formel (XIII) 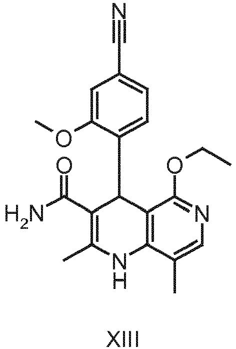 hergestellt wird, indem der Verbindung der Formel (XVIII) 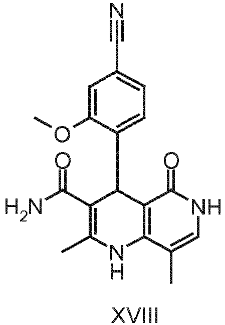 mit dem Orthoester (XX) 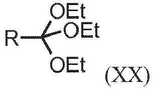 umgesetzt wird, wobei R für H oder Methyl stehen kann, und die Verbindung der Formel (XVIII) 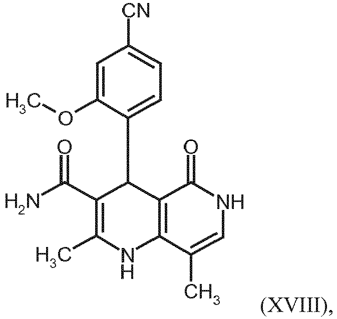 hergestellt wird, indem die Verbindungen der Formel (XVI a,b) 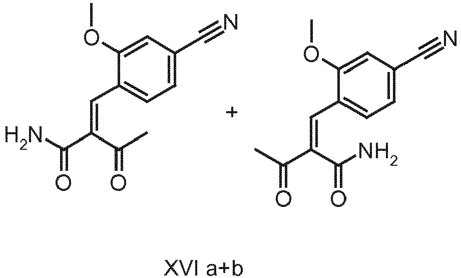 mit der Verbindung der Formel (IX) 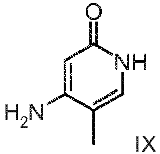 umgesetzt werden, und
die Verbindung der Formel (XVI a,b) hergestellt wird, indem die die Verbindung der Formel (VI) 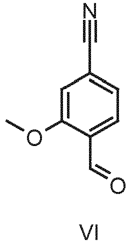 mit der Verbindung der Formel (XVII) 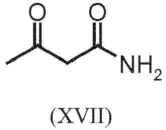 umgesetzt wird.

Ein weiterer Gegenstand der vorliegenden Erfindung ist ein Verfahren zur Herstellung der Verbindung der Formel (I) 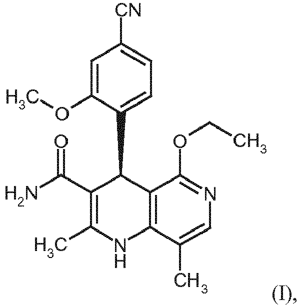 dadurch gekennzeichnet, dass die racemische Verbindung der Formel (XIII) in ihre Enantiomere getrennt wird, wobei die Verbindung der Formel (XIII) 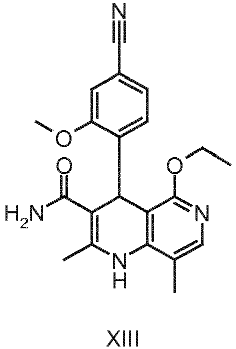 hergestellt wird, indem die Verbindung der Formel (XVIII) 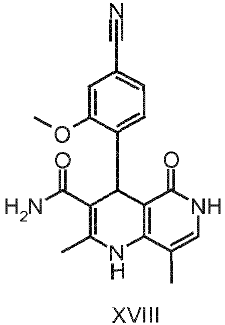 mit dem Orthoester (XX) 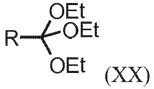 umgesetzt wird, wobei R für H oder Methyl stehen kann, und
die Verbindung der Formel (XVIII) 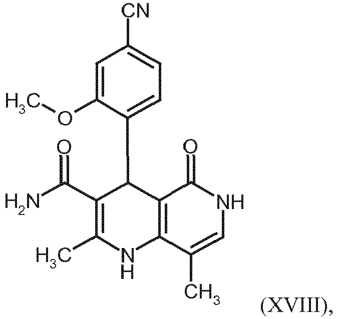 hergestellt wird, indem die Verbindungen der Formel (XVI a,b) 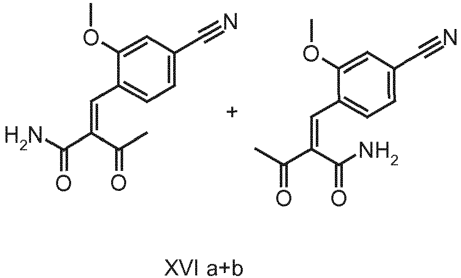 mit der Verbindung der Formel (IX) 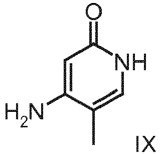 umgesetzt werden.

Ein weiterer Gegenstand der vorliegenden Erfindung ist ein Verfahren zur Herstellung der Verbindung der Formel (I) 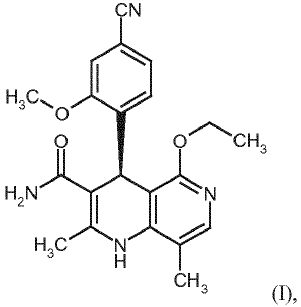 dadurch gekennzeichnet, dass die racemische Verbindung der Formel (XIII) in ihre Enantiomere getrennt wird, wobei die Verbindung der Formel (XIII) 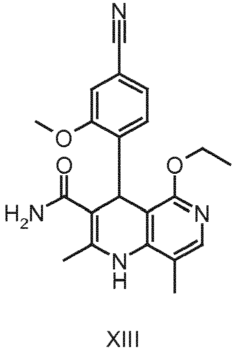 hergestellt wird, indem die Verbindung der Formel (XVIII) 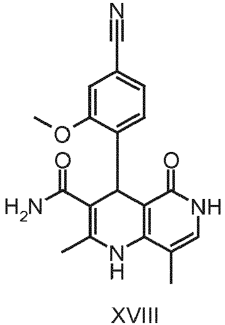 mit dem Orthoester (XX) 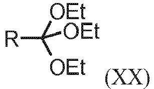 umgesetzt wird, wobei R für H oder Methyl stehen kann.

Ein weiterer Gegenstand der vorliegenden Erfindung ist ein Verfahren zur Herstellung der Verbindung der Formel (I) 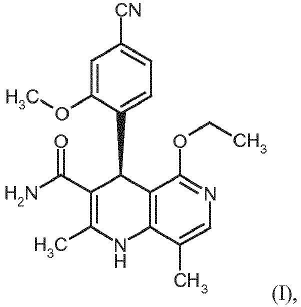 unter Verwendung der Verbindung der Formel (XVIII)

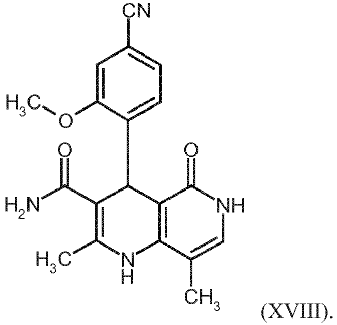

Ein weiterer Gegenstand der vorliegenden Erfindung ist ein Verfahren zur Herstellung der Verbindung der Formel (I) 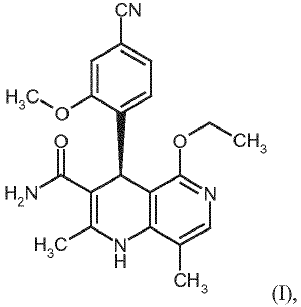 unter Verwendung der Verbindungen der Formel (XVI a,b)

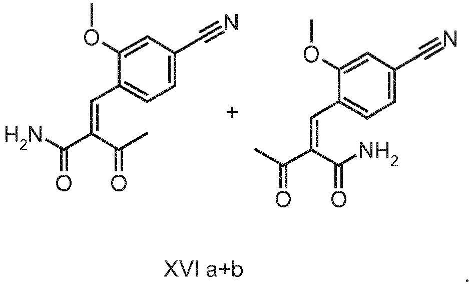

Ein weiterer Gegenstand der vorliegenden Erfindung ist ein Verfahren zur Herstellung der Verbindung der Formel (I) 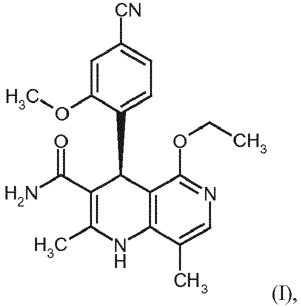 unter Verwendung der Verbindung der Formel (XVIII) 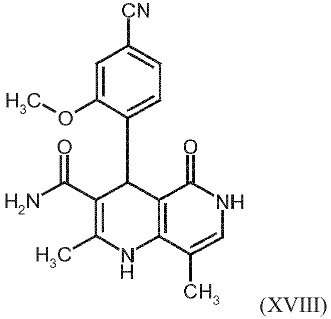 und der Verbindungen der Formel (XVI a,b)

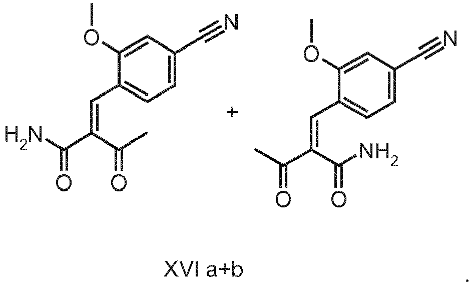

Ein weiterer Gegenstand der vorliegenden Erfindung ist eine Verbindung der Formel 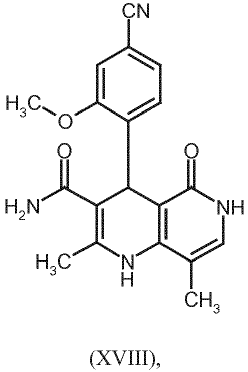 sowie dessen Salze, Solvate und Solvate der Salze.

Ein weiterer Gegenstand der vorliegenden Erfindung ist eine Verbindung der Formel (XVI a,b) als E/Z Gemisch 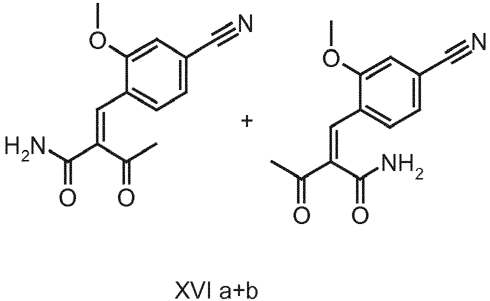 sowie dessen Salze, Solvate und Solvate der Salze.

Ein weiterer Gegenstand der vorliegenden Erfindung ist ein Verfahren zur Herstellung der Verbindung der Formel (XVIII) 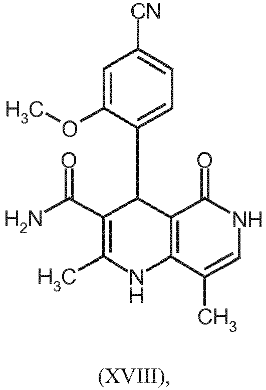 dadurch gekennzeichnet, dass die Verbindungen der Formel (XVI a,b) 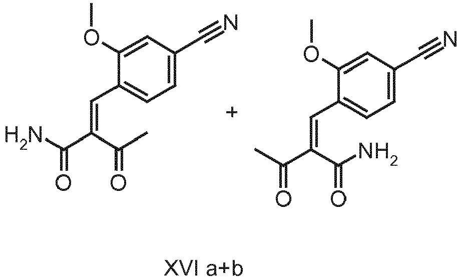 mit der Verbindung der Formel (IX) 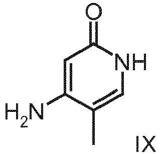 umgesetzt werden.

Ein weiterer Gegenstand der vorliegenden Erfindung ist ein Verfahren zur Herstellung der Verbindungen der Formel (XVI a,b) 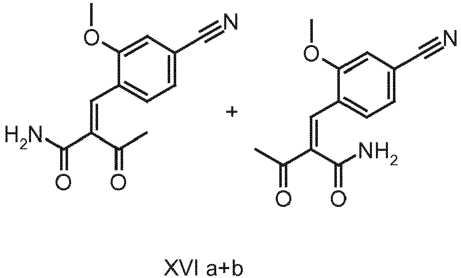 dadurch gekennzeichnet, dass die Verbindung der Formel (VI) 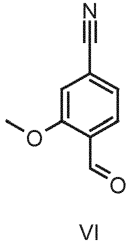 mit der Verbindung der Formel (XVII) 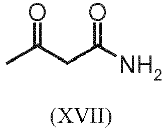 umgesetzt wird.

Ein weiterer Gegenstand der vorliegenden Erfindung ist ein Verfahren zur Herstellung der Verbindung (XIII) 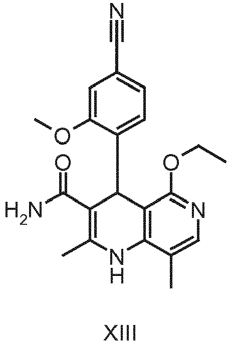 dadurch gekennzeichnet, dass die Verbindung der Formel (XVIII) 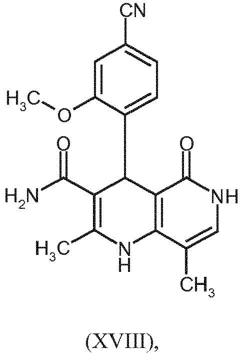 mit dem Orthoester (XX) 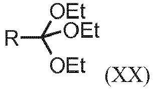 umgesetzt wird, wobei R für H oder Methyl stehen kann.

Ein weiterer Gegenstand der vorliegenden Erfindung ist ein Verfahren zur Herstellung der Verbindung der Formel (XIII) 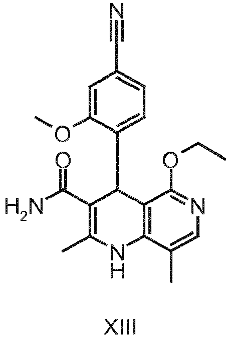 dadurch gekennzeichnet, dass die Verbindung der Formel (XVIII) 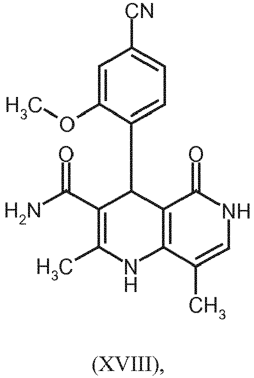 mit dem Orthoester (XX) 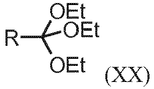 umgesetzt wird, wobei R für H oder Methyl stehen kann, und
dadurch gekennzeichnet, dass die Verbindung der (XVIII) hergestellt wird, indem die Verbindungen der Formel (XVI a,b) 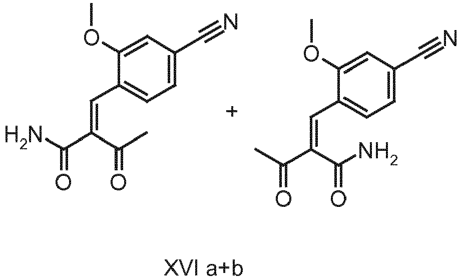 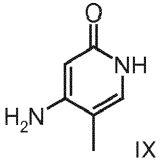 umgesetzt werden.

Ein weiterer Offenbarungsgegenstand ist ein Verfahren zur Herstellung der Verbindung der Formel (I) in kristalliner Modifikation I 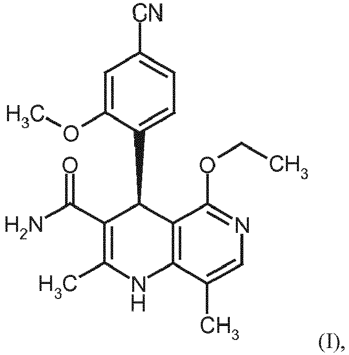 gemäß den oben beschriebenen Verfahren zur Herstellung der Verbindung der Formel (I), dadurch gekennzeichnet, das die Verbindung der Formel (I) vorliegend in einer oder mehreren Modifikationen oder als Solvat in einem inerten Lösungsmittel bei einer Temperatur von 20°C - 120°C gerührt und die Verbindung der Formel (I) in der kristallinen Modifikation I isoliert wird.

Die erfindungsgemäßen Verbindungen, die Verbindung der Formel (I) und die Verbindung der Formel (I) in kristalliner Form der Modifikation I (hier im Weiteren als die erfindungsgemäßen Verbindungen bezeichnet) wirken als Antagonisten des Mineralokorticoid-Rezeptors und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind geeignet zur Verwendung für die Prophylaxe und/oder Behandlung von verschiedenen Erkrankungen und krankheitsbedingten Zuständen, insbesondere von Erkrankungen, die entweder durch eine Erhöhung der Aldosteron-Konzentration im Plasma oder durch eine Veränderung der Aldosteron-Plasmakonzentration relativ zur Renin-Plasmakonzentration gekennzeichnet sind oder mit diesen Veränderungen einhergehen. Beispielsweise seien genannt: idiopathischer primärer Hyperaldosteronismus, Hyperaldosteronismus bei Nebennierenhyperplasie, Nebennierenadenomen und/oder Nebennierencarzinomen, Hyperaldosteronismus bei Leberzirrhose, Hyperaldosteronismus bei Herzinsuffizienz sowie (relativer) Hyperaldosteronismus bei essentieller Hypertonie.

Die erfindungsgemäßen Verbindungen sind aufgrund ihres Wirkmechanismus ferner geeignet zur Verwendung für die Prophylaxe des plötzlichen Herztodes bei Patienten, die unter einem erhöhten Risiko stehen, an einem plötzlichen Herztod zu versterben. Dies sind insbesondere Patienten, die z.B. an einer der folgenden Erkrankungen leiden: primäre und sekundäre Hypertonie, hypertensive Herzkrankheit mit oder ohne kongestive Herzinsuffizienz, therapieresistente Hypertonie, akute und chronische Herzinsuffizienz, koronare Herzerkrankung, stabile und instabile Angina pectoris, myokardiale Ischämie, Myokardinfarkt, dilatative Kardiomyopathien, angeborene primäre Kardiomyopathien wie z.B. Brugada-Syndrom, durch die Chagas-Erkrankung hervorgerufene Kardiomyopathien, Schock, Arteriosklerose, atriale und ventrikuläre Arrhythmie, transitorische und ischämische Attacken, Hirnschlag, entzündliche kardiovaskuläre Erkrankungen, periphere und kardiale Gefäßerkrankungen, periphere Durchblutungsstörungen, arterielle Verschlusskrankheiten wie Claudicatio intermittens, asymptomatische linksventrikuläre Dysfunktion, Myokarditis, hypertrophe Veränderungen des Herzens, pulmonale Hypertonie, Spasmen der Koronararterien und peripherer Arterien, Thrombosen, thromboembolische Erkrankungen sowie Vaskulitis.

Die erfindungsgemäßen Verbindungen können ferner verwendet werden für die Prophylaxe und/ oder Behandlung von Ödembildung, wie zum Beispiel pulmonales Ödem, renales Ödem oder Herzinsuffizienz-bedingtes Ödem, und von Restenosen, wie nach Thrombolysetherapien, percutan-transluminalen Angioplastien (PTA) und Koronarangioplastien (PTCA), Herztransplantationen sowie Bypass-Operationen.

Weiterhin eignen sich die erfindungsgemäßen Verbindungen zur Verwendung als kaliumsparendes Diuretikum und bei Elektrolytstörungen wie zum Beispiel Hyperkalzämie, Hypernatriämie oder Hypokaliämie.

Die erfindungsgemäßen Verbindungen eignen sich ebenso zur Verwendung in der Behandlung von Nierenerkrankungen, wie akutem und chronischem Nierenversagen, hypertensiver Nierenkrankheit, arteriosklerotischer Nephritis (chronisch und interstitiell), Nephrosklerose, chronischer Niereninsuffizienz und zystischen Nierenerkrankungen, zur Verhinderung von Nierenschäden, die zum Beispiel durch Immunsuppressiva wie Cyclosporin A bei Organtransplantationen hervorgerufen werden können, sowie bei Nierenkrebs.

Außerdem können die erfindungsgemäßen Verbindungen verwendet werden für die Prophylaxe und/oder Behandlung von Diabetes mellitus und diabetischen Folgeerkrankungen wie z.B. Neuropathie und Nephropathie.

Die erfindungsgemäßen Verbindungen können ferner verwendet werden für die Prophylaxe und/ oder Behandlung von Mikroalbuminurie, zum Beispiel bedingt durch Diabetes mellitus oder Bluthochdruck, sowie der Proteinurie.

Die erfindungsgemäßen Verbindungen eignen sich auch für die Verwendung in der Prophylaxe und/oder Behandlung von Erkrankungen, die entweder mit einer Erhöhung der Glukokortikoid-Konzentration im Plasma oder mit einer lokalen Konzentrationserhöhung von Glukokortikoiden im Gewebe (z.B. des Herzens) einhergehen. Beispielsweise seien genannt: Funktionsstörungen der Nebenniere, die zur Überproduktion von Glukokortikoiden führen (Cushing-Syndrom), Nebennierenrindentumore mit resultierender Überproduktion von Glukokortikoiden sowie Hypophysentumore, die autonom ACTH (adrenokortikotropes Hormon) produzieren und dadurch zu Nebennierenhyperplasien mit resultierendem Morbus Cushing führen.

Außerdem können die erfindungsgemäßen Verbindungen für die Prophylaxe und/oder Behandlung von Obesitas, des metabolischen Syndroms und der obstruktiven Schlaf-Apnoe verwendet werden. Die erfindungsgemäßen Verbindungen können ferner verwendet werden für die Prophylaxe und/ oder Behandlung von entzündlichen Erkrankungen, die z.B. durch Viren, Spirochäten, Pilze, Bakterien oder Mykobakterien hervorgerufen werden, sowie von entzündlichen Erkrankungen unbekannter Ätiologie, wie der Polyarthritis, dem Lupus erythematodes, der Peri- oder Polyarteriitis, der Dermatomyositis, der Sklerodermie und der Sarkoidose.

Weiterhin können die erfindungsgemäßen Verbindungen verwendet werden für die Behandlung von zentralnervösen Erkrankungen wie Depressionen, Angstzuständen und chronischen Schmerzen, insbesondere Migräne, sowie bei neurodegenerativen Erkrankungen wie der Alzheimer'schen Krankheit und dem Parkinson-Syndrom.

Die erfindungsgemäßen Verbindungen sind auch geeignet zur Verwendung für die Prophylaxe und/oder Behandlung von vaskulären Schäden, z.B. nach Interventionen wie percutan-transluminaler koronarer Angioplastie (PTCA), Implantationen von Stents, koronarer Angioskopie, Reokklusion oder Restenose nach Bypass-Operationen, sowie bei endothelialer Dysfunktion, bei Morbus Raynaud, bei der Thrombangiitis obliterans (Buerger-Syndrom) und beim Tinnitus-Syndrom.

Weiterer Offenbarungsgegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Offenbarungsegenstand ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Offenbarungsegenstand ist die Verwendung in einem Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Offenbarungsgegenstand der vorliegenden Erfindung sind Arzneimittel, enthaltend mindestens eine der erfindungsgemäßen Verbindungen und einen oder mehrere weitere Wirkstoffe, insbesondere zur Verwendung in der Behandlung und/oder Prävention der zuvor genannten Erkrankungen. Als geeignete Kombinationswirkstoffe seien beispielhaft und vorzugsweise genannt:
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker und Rho-Kinase-Inhibitoren;
- Diuretika, insbesondere Schleifendiuretika sowie Thiazide und Thiazid-ähnliche Diuretika;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugs-weise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussende Verbindungen, wie beispielhaft und vorzugsweise Etomoxir, Dichloracetat, Ranolazine oder Trimetazidine.

Bei einer bevorzugten Ausführungsform werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Rho-Kinase-Inhibitoren sowie der Diuretika verstanden.

Bei einer bevorzugten Ausführungsform werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform werden die erfindungsgemäßen Verbindungen in Kombination mit einem Renin-Inhibitor, wie beispielhaft und vorzugsweise Aliskiren, SPP-600, SPP-635, SPP-676, SPP-800 oder SPP-1148, verabreicht.

Bei einer bevorzugten Ausführungsform werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter antithrombotisch wirkenden Mitteln (Antithrombotika) werden vorzugsweise Verbindungen aus der Gruppe der Thrombozytenaggregationshemmer, der Antikoagulantien oder der profibrinolytischen Substanzen verstanden.

Bei einer bevorzugten Ausführungsform werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform werden die erfindungsgemäßen Verbindungen in Kombination mit Heparin oder einem low molecular weight (LMW)-Heparin-Derivat verabreicht.

Bei einer bevorzugten Ausführungsform werden die erfindungsgemäßen Verbindungen in Kombination mit einem Vitamin K-Antagonisten, wie beispielhaft und vorzugsweise Coumarin, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform werden die erfindungsgemäßen Verbindungen in Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform werden die erfindungsgemäßen Verbindungen in Kombination mit einem Lipase-Inhibitor, wie beispielhaft und vorzugsweise Orlistat, verabreicht.

Bei einer bevorzugten Ausführungsform werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Offenbarungsgegenstand sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht. Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Experimenteller Teil

### Abkürzungen und Akronyme:

MS : Masse aus Massenspektrometrie
HPLC: Hochleistungsflüssigkeitschromatographie
DMF : Dimethylformamid
Red-Al Lösung in Toluol : Natrium-bis-(2-methoxy-ethoxy)-aluminium-dihydrid in Toluol
THF : Tetrahydrofuran
Aqu. HCl: wässrige Salzsäure
DMAP: 4-(Dimethylamino)-pyridin

### Beispiele

### Beispiel 1

### Methyl-4-brom-2-methoxybenzoat (XV)

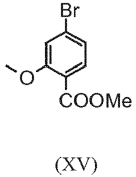

3,06 kg (22,12 mol) Kaliumcarbonat wurden in 3,6 1 Aceton vorgelegt und zum Rückfluss erhitzt. Zu dieser Suspension dosierte man 1,2 kg 4-Brom-2-hydroxybenzoesäure (5,53 mol), suspendierte in 7,8 1 Aceton zu und spülte mit 0,6 1 Aceton nach. Man erhitzte eine Stunde unter Rückfluss (starke Gasentwicklung!). Anschließend wurde unter Sieden 2,65 kg (21,01 mol) Dimethylsufat über 4 Stunden zudosiert. Anschließend wurde 2,5 Stunden unter Rückfluss nachgerührt. Man destillierte das Lösungsmittel weitgehend ab (bis zur Rührbarkeit) und gab 12 1 Toluol zu, anschließend wurde bei 110°C das restliche Aceton abdestilliert. Es wurden ca. 3 1 Destillat abdestilliert, diese wurden durch Zugabe von weiteren 3 1 Toluol zum Ansatz ergänzt. Man ließ auf 20°C abkühlen und gibt 10,8 1 Wasser dazu und rührte kräftig durch. Die organische Phase wurde abgetrennt und die Wasserphase nochmals mit 6,1 1 Toluol nachextrahiert. Die vereinigten organischen Phasen wurden mit 3 1 gesättigter Kochsalzlösung gewaschen und die Toluolphase bis auf ca. 4 1 eingeengt. Eine Gehaltsbestimmung durch Eindampfen einer Teilmenge ergab umgerechnet eine Ausbeute 1,306 kg (96,4 % der Theorie). Die Lösung wurde direkt in die Folgestufe eingesetzt.
HPLC-Methode A: RT ca. 11,9 min.
MS (EIpos): m/z = 245 [M+H]^{+ 1}H NMR (400 MHz, CD₂Cl₂): δ = 3.84 (s, 3H), 3.90 (s, 3H), 7.12-7.20 (m, 2H), 7.62 (d, 1H).

### Beispiel 2

### 4-Brom-2-methoxybenzaldehyd (XIX)

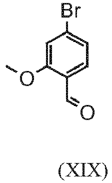

Man legte 1,936 kg (6,22 mol) 65%ige Red-A1 Lösung in Toluol mit 1,25 1 Toluol bei -5 °C vor. Zu dieser Lösung dosierte man 0,66 kg (6,59 mol) 1-Methylpiperazin zu und spült mit 150 ml Toluol nach, die Temperatur erhielt man dabei zwischen -7 bis -5°C. Man ließ 30 Minuten bei 0°C nachrühren. Diese Lösung dosierte man anschließend zu einer Lösung von 1,261 kg (5,147 mol) Methyl-4-brom-2-methoxybenzoat (XV), gelöst in 4 1 Toluol zu, die Temperatur hielt man bei - 8 bis 0°C. Man spülte zweimal mit 0,7 1 Toluol nach und rührte 1,5 Stunden bei 0°C nach. Zur Aufarbeitung dosierte man auf eine 0°C kalte wässrige Schwefelsäure (12,5 1 Wasser + 1,4 kg konz. Schwefelsäure). Die Temperatur sollte maximal auf 10°C steigen (langsame Dosierung). Der pH-Wert wurde ggf. durch Zugabe von weiterer Schwefelsäure auf pH 1 gestellt. Die organische Phase wurde abgetrennt und die wässrige Phase mit 7,6 1 Toluol nachextrahiert. Die vereinigten organischen Phasen wurden mit 5,11 Wasser gewaschen und anschließend weitgehend eingeengt und der Rückstand mit 10 1 DMF aufgenommen. Man engte erneut auf ca. 5 1 Volumen ein. Eine Gehaltsbestimmung durch Eindampfen einer Teilmenge ergab umgerechnet eine Ausbeute 1,041 kg (94,1 % der Theorie). Die Lösung wurde direkt in die Folgestufe eingesetzt.
HPLC-Methode A: RT ca. 12,1 min.
MS (EIpos): m/z = 162 [M+H]⁺
¹H-NMR (CDCl₃, 400MHz): δ = 3.93 (3H, s), 7.17 (2H, m), 7.68 (1H, d), 10.40 (1H, s)

### Beispiel 3

### 4-Formyl-3-methoxybenzonitril (VI)

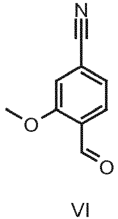

719 g (3,34 mol) 4-Brom-2-methoxybenzaldehyd (XVI) als Lösung in 4,5 1 DMF wurden mit 313 g (0,74 mol) Kaliumhexacyanoferrat (K₄[Fe(CN)₆]) und 354 g (3,34 mol) Natriumcarbonat und weiteren 1,2 1 DMF vorgelegt und 3,8 g (0,017 mol) Palladiumacetat wurde zugegeben. Man rührte 3 Stunden bei 120°C. Man ließ auf 20°C abkühlen und gab 5,7 1 Wasser zum Ansatz. Man extrahierte mit 171 Essigester und wusch die wässrige Phase nochmals mit 17 1 Essigester nach. Die organischen Phasen wurden vereinigt und weitgehend eingeengt mit 5 1 Isopropanol aufgenommen und auf ca. 2 1 eingeengt. Man erwärmte zum Sieden und tropfte 2 1 Wasser dazu. Man ließ auf 50 °C abkühlen und gab erneut 2 1 Wasser hinzu. Man kühlte auf 3°C ab und rührte eine Stunde bei dieser Temperatur. Das Produkt wurde abfiltriert und mit Wasser (2 mal 1,2 1) nachgewaschen. Man trocknete bei 40°C im Vakuum.
Ausbeute: 469 g (87 % d. Theorie) eines beigefarbenen Feststoffes.
HPLC-Methode A: RT ca. 8,3 min.
MS (EIpos): m/z = 162 [M+H]+
1H-NMR (300 MHz, DMSO-d6): δ = 3.98 (s, 3H), 7.53 (d, 1H), 7.80 (s, 1H), 7.81 (d, 1H), 10.37 (s, 1H).

### Beispiel 4

### (2E/2Z)-2-(4-Cyan-2-methoxybenzyliden)-3-oxobutanamid (XVI a,b)

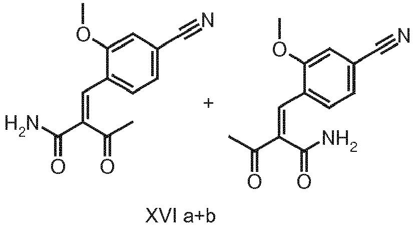

1000 g (6204,95 mmol) 4-Formyl-3-methoxybenzonitril (VI), 721,5 g (7135,7 mmol) 3-Oxobutanamid (XVII), 53g (620 mmol) Piperidin und 37,3 g (620 mmol) Eisessig wurden in 151 Dichlormethan 4 Stunden unter Rückfluss am Wasserabscheider erhitzt. Anschließend destillierte man ca. 10 1 Dichlormethan ab und ließ auf Raumtemperatur abkühlen. Man kühlte auf 0°C ab, ließ 4 Stunden bei dieser Temperatur nachrühren und filtrierte das Produkt ab, wusch 2 mal mit je 1000 ml kalten Dichlormethan nach. Es wurde bei 40°C im Vakuum unter Schleppgas getrocknet.
Ausbeute: 1439,8 g (95,0% der Theorie) eines gelbfarbigen Feststoffes.
HPLC-Methode A: RT ca. 3.55 min.
MS (EIpos): m/z = 245 [M+H]⁺
¹H-NMR (500 MHz, DMSO-d₆): δ = 2.35 (s, 3H), 3.30 (s, 2H), 3.90 (s, 3H), 7.45 (d, 1H), 7,7 (m, 3H), 7.75 (d, 1H), 8.85 (d, 1H)

### Beispiel 5

### 4-(4-Cyan-2-methoxyphenyl)-2,8-dimethyl-5-oxo-1,4,5,6-tetrahydro-1,6-naphthyridin-3-carboxamid (XVIII)

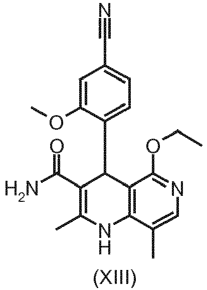

2,128 kg (8,712 mol) (2E/2Z)-2-(4-Cyan-2-methoxybenzyliden)-3-oxobutanamid (XVI a,b) wurden mit 29 1 2-Butanol aufgenommen und 1,277 kg (7,92 mol) 4-Amino-5-methylpyridon zugegeben und anschließend 12 h bei 120°C Innentemperatur unter Überdruck im geschlossenen Kessel erhitzt. Man kühlte über eine Rampe innerhalb von 5 h auf 0°C ab rührte 3 Stunden bei 0°C nach. Man filtrierte ab und wusch mit 2,1 1 kaltem Isopropanol nach. Man trocknete im Vakuum bei 60°C.

Ausbeute: 2,081 kg (75 % der Theorie bezogen auf 4-Amino-5-methylpyridon, da diese Komponente im Unterschuss eingesetzt wird) eines leicht gelbfarbigen Feststoffes.
HPLC-Methode A: RT ca. 3.64 min.
MS (EIpos): m/z = 351 [M+H]⁺
¹H-NMR (500 MHz, DMSO-d₆): δ = 2.00 (s, 3H), 2.10 (s, 3H), 3.78 (s, 3H), 5.22 (s, 1H), 6.65 (s(breit), 1H), 6.85 (s(breit), 1H), 6.91 (s, 1H), 7.11 (d, 1H), 7.28 (d, 1H), 7.35 (s, 1H), 7.52 (s, 1H), 10,61 (s, 1H)

### Beispiel 5

### 4-(4-Cyano-2-methoxyphenyl)-5-ethoxy-2,8-dimethyl-1,4-dihydro-1,6-naphthyridin-3-carboxamid (XIII)

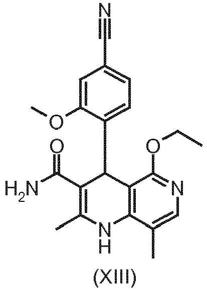

1,857 kg (5,3 mol) 4-(4-Cyan-2-methoxyphenyl)-2,8-dimethyl-5-oxo-1,4,5,6-tetrahydro-1,6-naphthyridin-3-carboxamid (XVIII) und 4,70 kg (29 mol) Orthoessigsäuretriethylester wurden in 12,15 1 Dimethylacetamid gelöst und 157,5 g konzentrierter Schwefelsäure zugegeben. Man erwärmte 1,5 Stunden bei 115°C und kühlte anschließend auf 50°C ab. Bei 50°C wurden über 30 Minuten 12,15 1 Wasser zu getropft. Nach beendeter Zugabe wurde mit 10 g der Titelbelbindung (XI) angeimpft und weitere 12,15 1 Wasser über 30 Minuten bei 50°C zugetropft. Man kühlte auf 0°C (Rampe, 2 Stunden) ab und rührte 2 Stunden bei 0°C nach. Das Produkt wurde abfiltriert, 2 mal mit je 7,71 Wasser gewaschen und im Vakuum bei 50°C getrocknet.
Ausbeute: 1,845 kg (92,0 % der Theorie) eines leicht gelbfarbigen Feststoffes.
HPLC-Methode B: RT ca. 10,2 min.
MS (EIpos): m/z = 433 [M+H]⁺
¹H-NMR (300 MHz, DMSO-d₆): δ = 1.11 (t, 3H), 2.16 (s, 3H), 2.42 (s, 3H), 2.78 (m, 2H), 3.77 (s, 3H), 4.01-4.13 (m, 4H), 5.37 (s, 1H), 7.25 (d, 1H), 7.28-7.33 (m, 2H), 7.60 (s, 1H), 8.35 (s, 1H).

### Beispiel 6

### (4S)-4-(4-Cyano-2-methoxyphenyl)-5-ethoxy-2,8-dimethyl-1,4-dihydro-1,6-naphthyridin-3-carboxamid (I) als Lösung in Acetonitril/Methanol 40:60

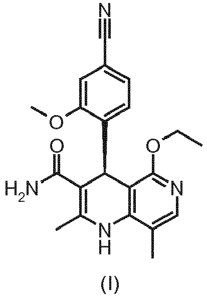

### Enantiomeren-Trennung auf einer SMB-Anlage

Als Feed-Lösung diente eine Lösung entsprechend einer Konzentration bestehend aus 50 g Racemat 4-(4-Cyano-2-methoxyphenyl)-5-ethoxy-2,8-dimethyl-1,4-dihydro-1,6-naphthyridin-3-carbox-amid (XIII), gelöst in 1 Liter einer Mischung aus Methanol/Acetonitril 60:40.

Es wurde über eine SMB-Anlage an einer stationären Phase: Chiralpak AS-V, 20 µm chromatographiert. Der Druck betrug 30 bar, als Eluent wird eine Mischung aus Methanol/Acetonitril 60:40 verwendet.

9,00 kg 4-(4-Cyano-2-methoxyphenyl)-5-ethoxy-2,8-dimethyl-1,4-dihydro-1,6-naphthyridin-3-carbox-amid (XIII) wurden in 180 1 einer Mischung bestehend aus Methanol/Acetonitril 60:40 gelöst und mittels SMB chromatographiert. Nach aufkonzentrieren der produkthaltigen Fraktionen erhielt man 69,68 liter einer 6,2 %igen Lösung (entspricht 4,32 kg (4S)- 4-(4-Cyano-2-methoxyphenyl)-5-ethoxy-2,8-dimethyl-1,4-dihydro-1,6-naphthyridin-3-carboxamid (I) als Lösung in Acetonitril/Methanol 40:60).
Ausbeute: 4,32 kg (48 % d. Theorie), gelöst in 69,68 liter Acetonitril/Methanol 40:60 als farblose Fraktion.
Enantiomerenreinheit: > 98,5% e.e. (HPLC, Methode D)
Eine Probe wird im Vakuum eingeengt und ergibt: MS (EIpos): m/z = 379 [M+H]⁺
¹H-NMR (300 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).

### Beispiel 7

### (4S)-4-(4-Cyano-2-methoxyphenyl)-5-ethoxy-2,8-dimethyl-1,4-dihydro-1,6-naphthyridin-3-carboxamid (I)

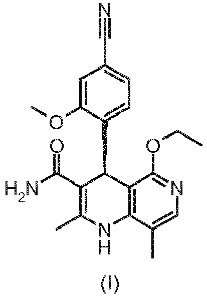

### Kristallisation und Polymorphie-Einstellung

64,52 Liter einer 6,2 %igen Lösung aus Beispiel 6 in einer Mischung Acetonitiril/Methanol 40:60 (entspricht 4,00 kg Verbindung I) wurden über eine Filterkerze (1,2 um) filtriert und anschließend bei 250 mbar soweit eingeengt, sodaß die Lösung noch rührbar war. Man fügte 481 Ethanol, vergällt mit Toluol zu und destillierte erneut bei 250 mbar bis zur Rührbarkeitsgrenze ab (Umdestillation auf Ethanol). Man gab weitere 48 1 Ethanol, vergällt mit Toluol zu und destillierte anschließend bei Normaldruck bis auf ein Gesamtvolumen von ca. 14 1 ab (Manteltemperatur 98°C). Man kühlte über eine Rampe (4 Stunden) auf 0°C ab, rührte 2 Stunden bei 0°C nach und filtrierte das Produkt ab. Es wurde zweimal mit je 4 1 kaltem Ethanol nachgewaschen und anschließend im Vakuum bei 50°C getrocknet.
Ausbeute: 3,64 kg (91 % d. Theorie) eines farblosen kristallinen Pulvers
Enantiomerenreinheit: >> 99 % e.e. (HPLC-Methode D); Retentionszeiten/RRT: (4S)- 4-(4-Cyano-2-methoxyphenyl)-5-ethoxy-2,8-dimethyl-1,4-dihydro-1,6-naphthyridin-3-carbox-amid (I) ca. 11 min. RRT: 1,00 ; (4R)- 4-(4-Cyano-2-methoxyphenyl)-5-ethoxy-2,8-dimethyl-1,4-dihydro-1,6-naphthyridin-3-carbox-amid (I) ca. 9 min. RRT: 0,82
Reinheit: > 99,8% (HPLC-Methode B), RT: ca. 6,7 min.
Gehalt: 99,9 % (gegen externen Standard)
spezifischer Drehwert (Chloroform, 589 nm, 19.7°C, c = 0.38600 g / 100 ml): - 148.8°.
MS (EIpos): m/z = 379 [M+H]⁺
¹H-NMR (300 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m, 2H), 7.14 (d, 1H), 7.28 (dd, 1H), 7.37 (d, 1H), 7.55 (s, 1H), 7.69 (s, 1H).
Schmelzpunkt: 252 °C (Verbindung der Formel (I) in kristalliner Form der Modifikation I)

### Physikochemische Charakterisierung von Verbindung der Formel (I) in kristalliner Form der Modifikation I

Verbindung der Formel (I) in kristalliner Form der Modifikation I schmilzt bei 252°C, ΔH = 95 - 113 Jg⁻¹ (Heizrate 20 Kmin⁻¹).

Eine Depression des Schmelzpunktes wurde in Abhängigkeit der Heizrate beobachtet.

Der Schmelzpunkt sinkt bei einer geringeren Heizrate (z.B. 2 Kmin⁻¹) weil Zersetzung eintritt.

Es wurden keine weiteren Phasenübergänge beobachtet. Ein Massenverlust von ca. 0,1 % wurde bis zu einer Temperatur von 175°C beobachtet.

### Stabilität und Feuchtigkeits-Einlagerung

Proben von Verbindung der Formel (I) in kristalliner Form der Modifikation I wurden bei 85 % and 97 % rel. Luftfeuchtigkeit (25°C) eingelagert. Die Proben wurden nach 12 Monaten mittels DSC, TGA und XRPD ausgewertet. Nach 12 Monaten wird in beiden Fällen eine Massenänderung von < 0,1 % festgestellt. D.h. Verbindung der Formel (I) in kristalliner Form der Modifikation I zeigt keine signifikante Wasserabsorption unter diesen Lagerbedingungen. Laut DSC, TGA und XRPD besteht kein Unterschied zu Verbindung der Formel (I) in kristalliner Form der Modifikation I.

### HPLC-Bedingungen/Methoden

### Methode A

YMC Hydrosphere C18
150*4,6 mm, 3,0 µm
25°C, 1 ml/min , 270 nm, 4 nm
0' : 70% TFA 0,1%*; 30% Acetonitril
17': 20% TFA 0,1% ; 80% Acetonitril
18': 70% TFA 0,1 %; 30% Acetonitril
*: TFA in Wasser

### Methode B

YMC Hydrosphere C18
150*4,6 mm, 3,0 µm
25°C, 1 ml/min , 255 nm, 6 nm
0' : 90% TFA 0,1%; 10% Acetonitril
20': 10% TFA 0,1% ; 90% Acetonitril
18': 10% TFA 0,1 %; 90% Acetonitril

### Methode C

Nucleodur Gravity C18
150*2 mm, 3,0 µm
35°C, 0,22 ml/min. , 255 nm, 6 nm
Lösung A: 0,58 g Ammoniumhydrogenphosphat und 0,66 g Ammoniumdihydrogenphosphat in 1 L Wasser (Ammoniumphosphat-Puffer pH 7,2)
Lösung B: Acetonitril
0' : 30% B ; 70% A
15' : 80% B ; 20% A
25' : 80% B ; 20% A

### Methode D

Säulen-Länge: 25 cm
Innendurchmesser: 4,6 mm
Füllung: Chiralpak IA, 5 µm
Reagenzien: 1. Acetonitril, für die HPLC
2. Methyl-tert-butyl-ether (MTBE), p.a.
Prüflösung Die Probe wird in einer Konzentration von 1,0 mg/mL
in Acetonitril gelöst.
(z. B. ca. 25 mg Probe, genau gewogen, in Acetonitril
zu 25,0 mL lösen.)
Elutionsmittel A. Acetonitril
B. Methyl-tert-butyl-ether (MTBE), p.a.
Durchflussrate 0,8 mL/min
Temperatur des Säulenofens 25 °C
Detektion Messwellenlänge: 255 nm
Bandbreite: 6 nm
Injektionsvolumen 5 µL
Zusammensetzung des Eluenten A und B im Volumenverhältnis 90:10 mischen.
Laufzeit des Chromatogramms 30 min
Retentionszeiten/RRT:
   (4S)- 4-(4-Cyano-2-methoxyphenyl)-5-ethoxy-2,8-dimethyl-1,4-dihydro-1,6-naphthyridin-3-carbox-amid (1) ca. 11 min. RRT: 1,00
   (4R)- 4-(4-Cyano-2-methoxyphenyl)-5-ethoxy-2,8-dimethyl-1,4-dihydro-1,6-naphthyridin-3-carbox-amid (1) ca. 9 min. RRT: 0,82

### Messparamater der Röntgendiffraktometrie für die Messung der Verbindung der Formel (I) in kristalliner Form der Modifikation I:

| | |
|---|---|
| Datensatz Name | 2429-08a r2 |
| Scan-Achse | 2Theta-Omega |
| Startposition [°2Th.] | 2,0000 |
| Endposition [°2Th.] | 37,9900 |
| Art der Divergenzblende | Fest |
| Größe der Divergenzblende [°] | 1,0000 |
| Temperatur der Messung [°C] | 25 |
| Anodenmaterial | Cu |
| K-Alphal [Å] | 1,54060 |
| Generatoreinstellung | 35 mA, 45 kV |
| Diffraktometer Typ | Transmission diffractometer |
| Goniometer Radius [mm] | 240,00 |
| Abstand Focus-Div.blende [mm] | 91,00 |
| Primärstrahl-Monochromator | Ja |
| Probendrehung | Ja |

| Peakmaximum [2 Theta] |
|---|
| Modifikation I |
| 8.5 |
| 11.4 |
| 11.9 |
| 13.4 |
| 14.1 |
| 14.8 |
| 15.0 |
| 15.4 |
| 16.0 |
| 17.2 |
| 18.5 |
| 19.0 |
| 19.8 |
| 20.5 |
| 20.8 |
| 22.1 |
| 22.7 |
| 23.0 |
| 23.1 |
| 23.6 |
| 23.9 |
| 24.6 |
| 24.9 |
| 25.2 |
| 25.6 |
| 26.0 |
| 26.5 |
| 27.1 |
| 27.3 |
| 28.3 |
| 28.5 |
| 28.8 |
| 29.6 |
| 30.1 |
| 30.6 |
| 31.5 |
| 31.9 |
| 32.4 |
| 32.9 |
| 33.1 |
| 33.4 |
| 33.7 |
| 34.5 |
| 34.7 |
| 35.0 |
| 35.8 |
| 36.2 |
| 36.5 |
| 37.2 |
| 37.4 |

### Messbedingungen für die IR- und Ramanspektroskopie für die Messung der Verbindung der Formel (I) in kristalliner Form der Modifikation I:

| IR: | |
|---|---|
| Gerät | Perkin Elmer Spectrum One |
| Anzahl Scans | 32 |
| Auflösung | 4 cm⁻¹ |
| Technik | Diamant ATR-Einheit |

| Raman: | |
|---|---|
| Gerät | Bruker Raman RFS 100/S |
| Anzahl Scans | 64 |
| Auflösung | 2 - 4 cm⁻¹ |
| Laser Power | 350 mW |
| Laser Wellenlänge | 1064 nm |

| Bandenmaximum [cm⁻¹] | |
|---|---|
| IR-ATR | Raman |
| Modifikation I | Modifikation 1 |
| 3475 | 3074 |
| 3416 | 2997 |
| 3366 | 2970 |
| 3074 | 2941 |
| 2992 | 2920 |
| 2952 | 2836 |
| 2835 | 2231 |
| 2230 | 1659 |
| 1681 | 1641 |
| 1658 | 1623 |
| 1606 | 1601 |
| 1572 | 1577 |
| 1485 | 1487 |
| 1464 | 1443 |
| 1454 | 1383 |
| 1431 | 1362 |
| 1420 | 1327 |
| 1407 | 1303 |
| 1381 | 1267 |
| 1355 | 1230 |
| 1341 | 1191 |
| 1325 | 1161 |
| 1303 | 1123 |
| 1285 | 1093 |
| 1267 | 1032 |
| 1255 | 991 |
| 1229 | 883 |
| 1222 | 827 |
| 1161 | 810 |
| 1136 | 759 |
| 1097 | 734 |
| 1031 | 708 |
| 991 | 671 |
| 976 | 613 |
| 967 | 528 |
| 924 | 505 |
| 909 | 471 |
| 875 | 442 |
| 847 | 346 |
| 827 | 320 |
| 810 | 297 |
| 776 | 186 |
| 758 | 155 |
| 746 | 114 |
| 733 | |
| 723 | |
| 706 | |
| 697 | |
| 670 | |

## Patentansprüche

1. Verfahren zur Herstellung der Verbindung der Formel (I) 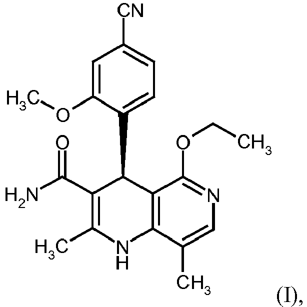 **dadurch gekennzeichnet, dass** die racemische Verbindung der Formel (XIII) in ihre Enantionmere getrennt wird, und die Verbindung der Formel (XIII) 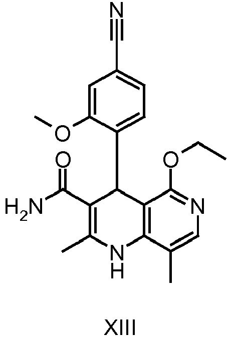 hergestellt wird, indem die Verbindung der Formel (XVIII) 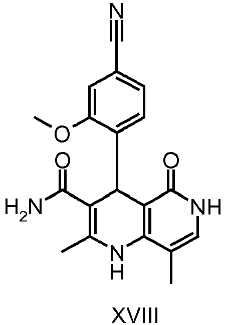 mit dem Orthoester (XX) 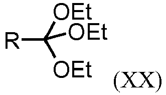 umgesetzt wird, wobei R für H oder Methyl stehen kann, und
die Verbindung der Formel (XVIII) 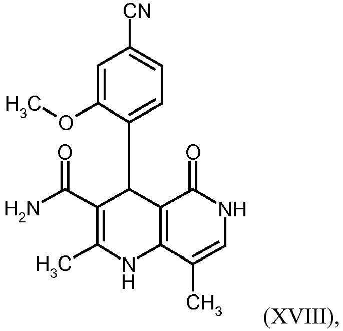 hergestellt wird, indem die Verbindungen der Formel (XVI a,b) 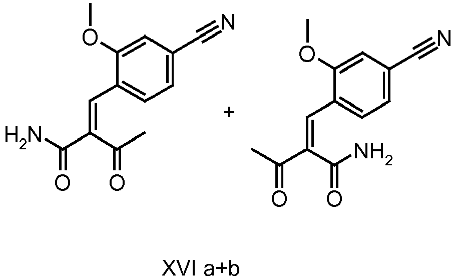 mit der Verbindung der Formel (IX) 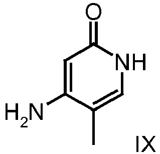 umgesetzt werden, und
die Verbindung der Formel (XVI a,b) hergestellt wird, indem die Verbindung der Formel (VI) 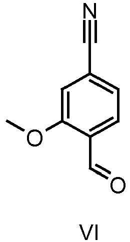 mit der Verbindung der Formel (XVII) 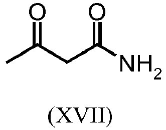 umgesetzt wird.

2. Verfahren zur Herstellung der Verbindung der Formel (I) 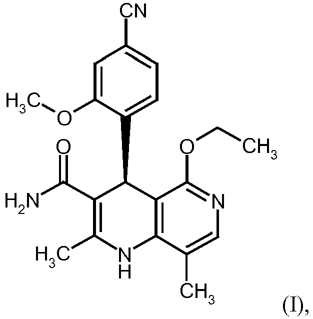 **dadurch gekennzeichnet, dass** die racemische Verbindung der Formel (XIII) in ihre Enantiomere getrennt wird, wobei die Verbindung der Formel (XIII) 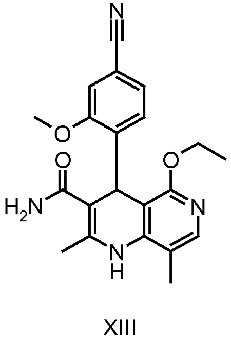 hergestellt wird, indem die Verbindung der Formel (XVIII) 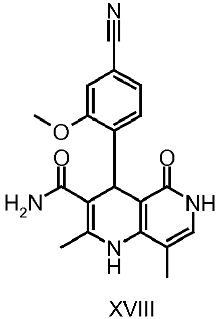 mit dem Orthoester (XX) 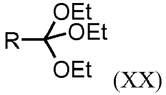 umgesetzt wird, wobei R für H oder Methyl stehen kann, und
die Verbindung der Formel (XVIII) 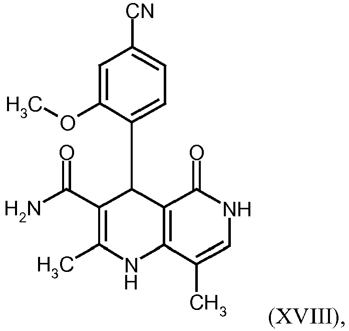 hergestellt wird, indem die Verbindungen der Formel (XVI a,b) 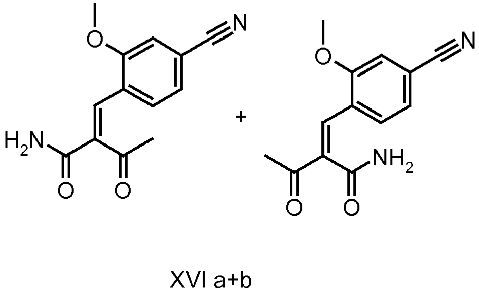 mit der Verbindung der Formel (IX) 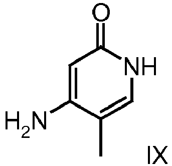 umgesetzt werden.

3. Verfahren zur Herstellung der Verbindung der Formel (I) 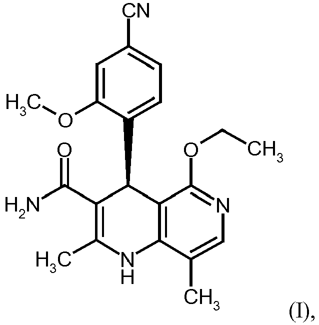 **dadurch gekennzeichnet, dass** die racemische Verbindung der Formel (XIII) in ihre Enantiomere getrennt wird, wobei die Verbindung der Formel (XIII) 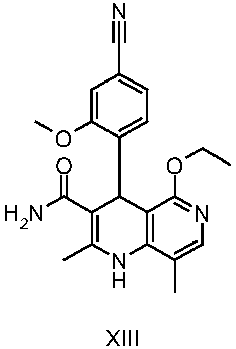 hergestellt wird, indem die Verbindung der Formel (XVIII) 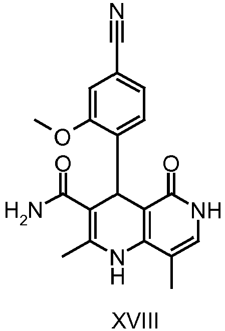 mit dem Orthoester (XX) 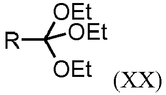 umgesetzt wird, wobei R für H oder Methyl stehen kann.

4. Verfahren zur Herstellung der Verbindung der Formel (I) 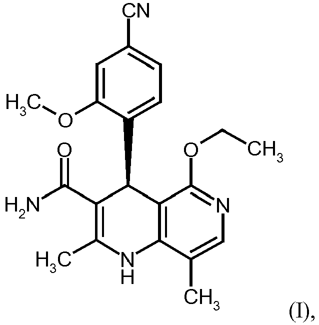 unter Verwendung der Verbindung der Formel (XVIII)

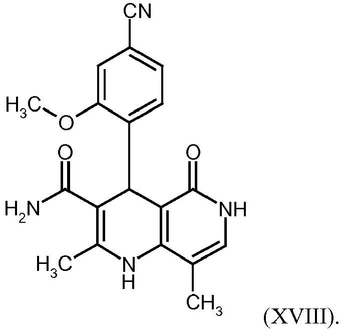

5. Verfahren zur Herstellung der Verbindung der Formel (I) 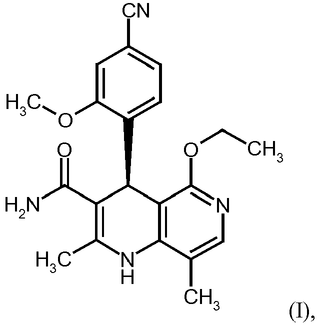 unter Verwendung der Verbindungen der Formel (XVI a,b)

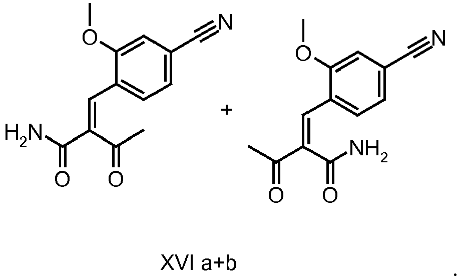

6. Verfahren zur Herstellung der Verbindung der Formel (I) 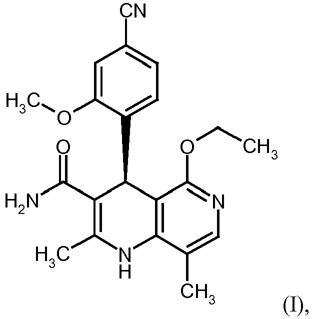 unter Verwendung der Verbindung der Formel (XVIII) 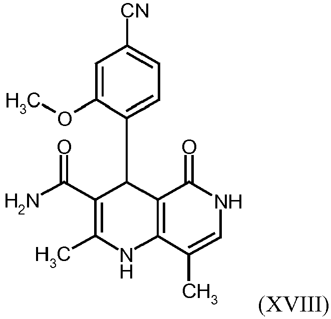 und
der Verbindungen der Formel (XVI a,b)

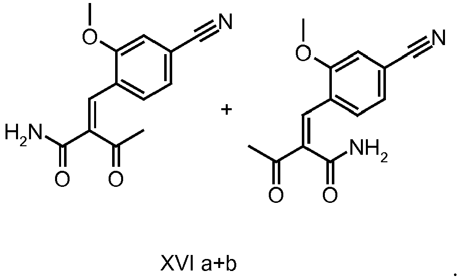

7. Verbindung der Formel 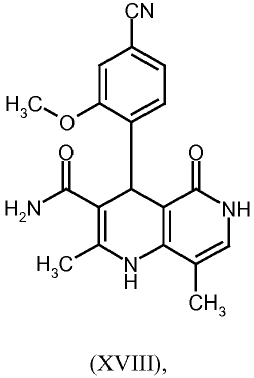 sowie dessen Salze, Solvate und Solvate der Salze.

8. Verbindung der Formel (XVI a+b) als E/Z Gemisch 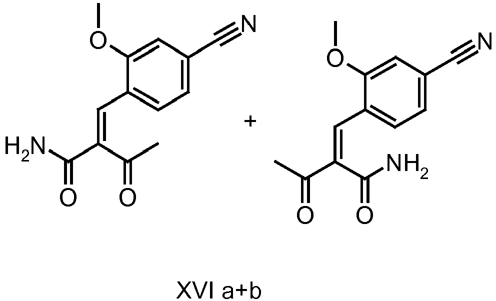 sowie dessen Salze, Solvate und Solvate der Salze.

9. Verfahren zur Herstellung der Verbindung der Formel (XVIII) 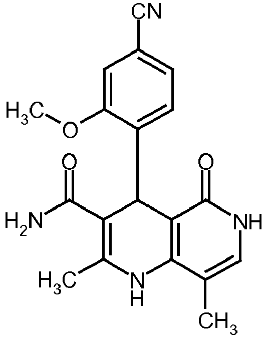 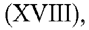 **dadurch gekennzeichnet, dass** die Verbindungen der Formel (XVI a,b) 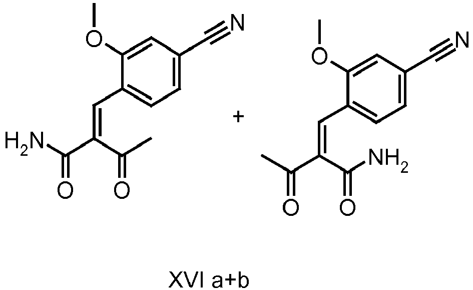 mit der Verbindung der Formel (IX) 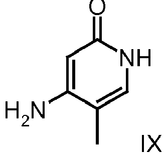 umgesetzt werden.

10. Verfahren zur Herstellung der Verbindungen der Formel (XVI a,b) 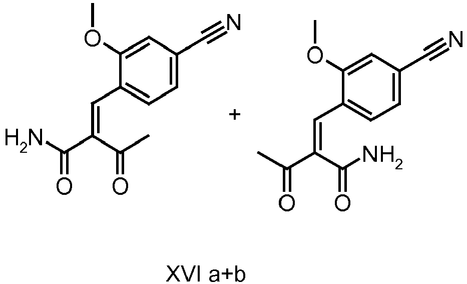 **dadurch gekennzeichnet, dass** die Verbindung der Formel (VI) 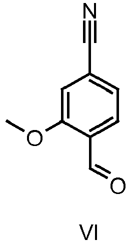 mit der Verbindung der Formel (XVII) 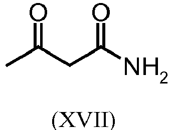 umgesetzt wird.

11. Verfahren zur Herstellung der Verbindung (XIII) 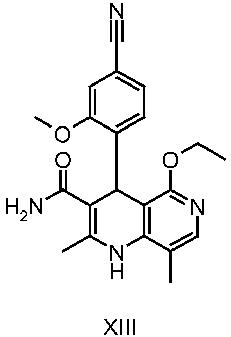 **dadurch gekennzeichnet, dass** die Verbindung der Formel (XVIII) 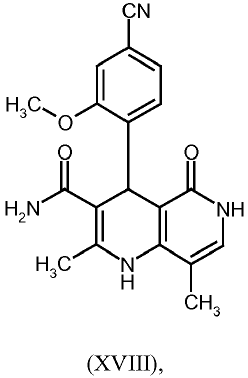 mit dem Orthoester (XX) 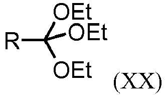 umgesetzt wird, wobei R für H oder Methyl stehen kann.

12. Verfahren zur Herstellung der Verbindung der Formel (XIII) 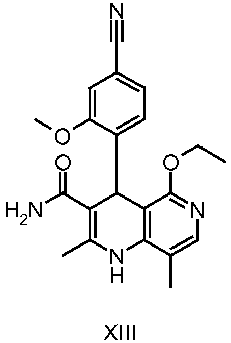 **dadurch gekennzeichnet, dass** die Verbindung der Formel (XVIII) 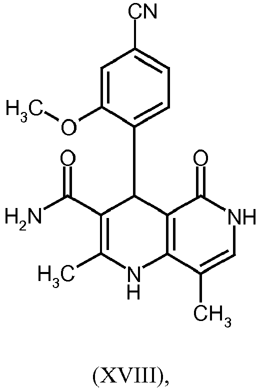 mit dem Orthoester (XX) 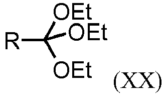 umgesetzt wird, wobei R für H oder Methyl stehen kann, und
**dadurch gekennzeichnet, dass** die Verbindung der (XVIII) hergestellt wird, indem die Verbindungen der Formel (XVI a,b) 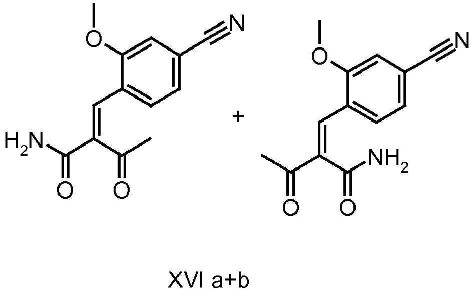 mit der Verbindung der Formel (IX) 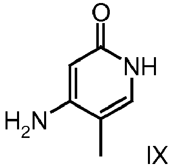 umgesetzt werden.

## Claims

1. Process for preparing the compound of the formula (I) 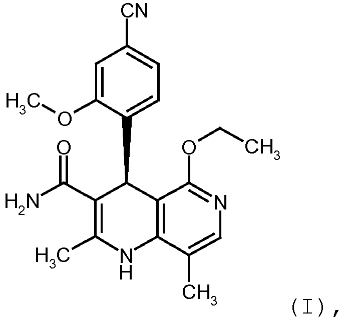 **characterized in that** the racemic compound of the formula (XIII) is separated into its enantiomers, and the compound of the formula (XIII) 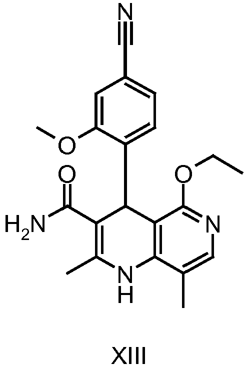 is prepared by reacting the compound of the formula (XVIII) 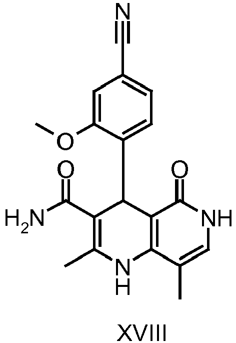 with the orthoester (XX) 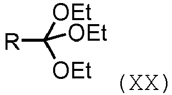 where R may be H or methyl, and the compound of the formula (XVIII) 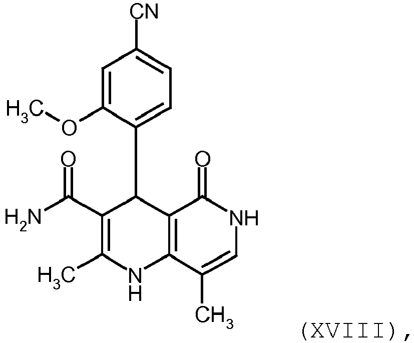 is prepared by reacting the compounds of the formula (XVI a,b) 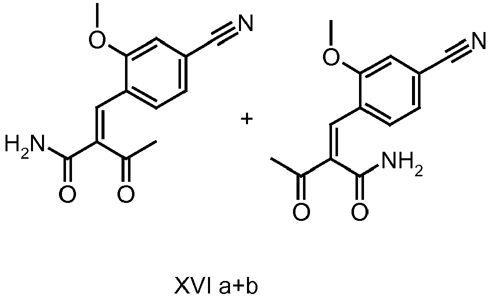 with the compound of the formula (IX) 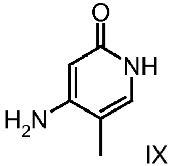 and
the compound of the formula (XVI a,b) is prepared by reacting the compound of the formula (VI) 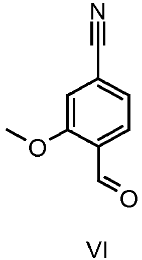 with the compound of the formula (XVII)

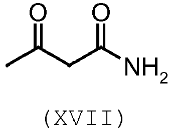

2. Process for preparing the compound of the formula (I) 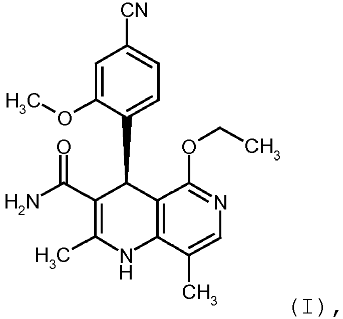 **characterized in that** the racemic compound of the formula (XIII) is separated into its enantiomers, where the compound of the formula (XIII) 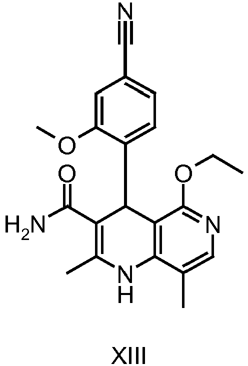 is prepared by reacting the compound of the formula (XVIII) 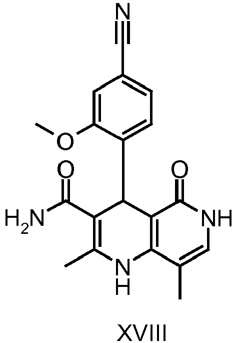 with the orthoester (XX) 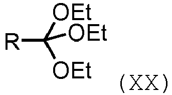 where R may be H or methyl, and
the compound of the formula (XVIII) 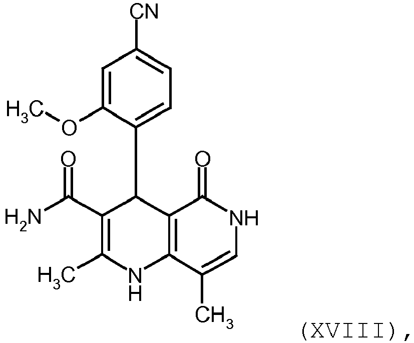 is prepared by reacting the compounds of the formula (XVI a,b) 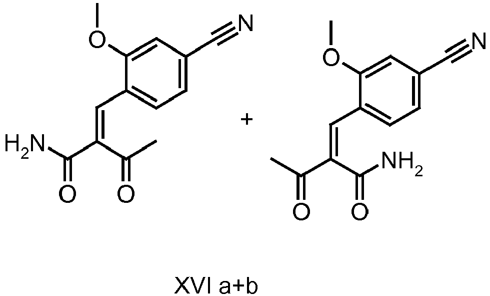 with the compound of the formula (IX)

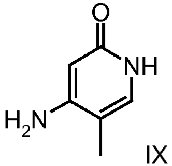

3. Process for preparing the compound of the formula (I) 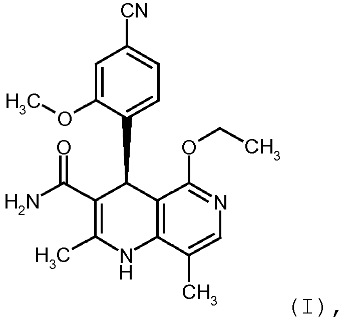 **characterized in that** the racemic compound of the formula (XIII) is separated into its enantiomers, where the compound of the formula (XIII) 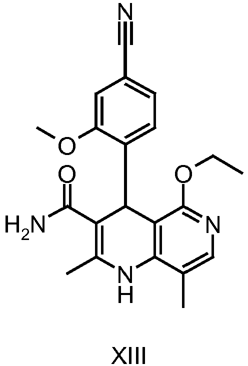 is prepared by reacting the compound of the formula (XVIII) 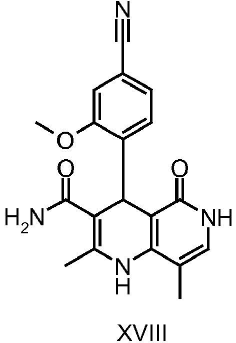 with the orthoester (XX) 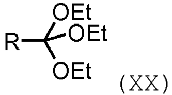 where R may be H or methyl.

4. Process for preparing the compound of the formula (I) 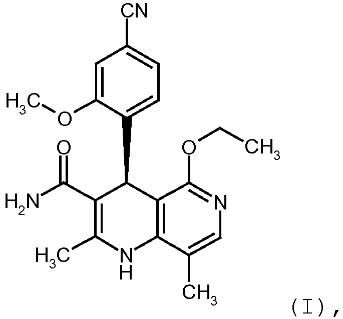 using the compound of the formula (XVIII)

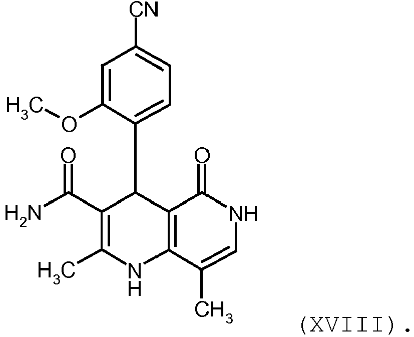

5. Process for preparing the compound of the formula (I) 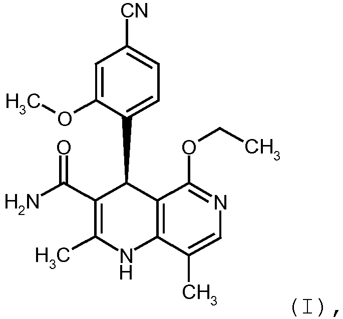 using the compounds of the formula (XVI a,b)

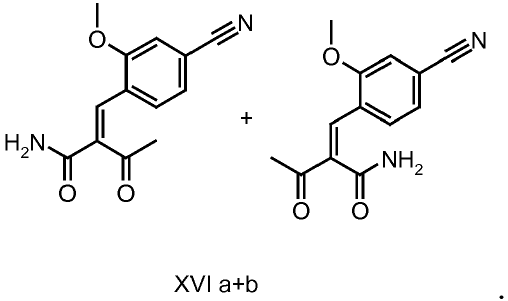

6. Process for preparing the compound of the formula (I) 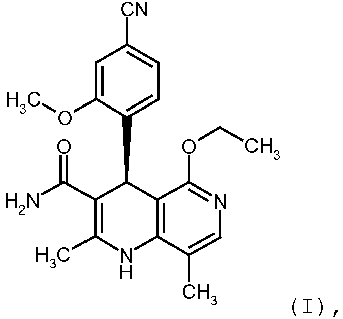 using the compound of the formula (XVIII) 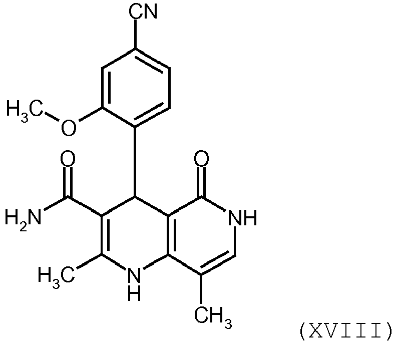 and
the compounds of the formula (XVI a,b)

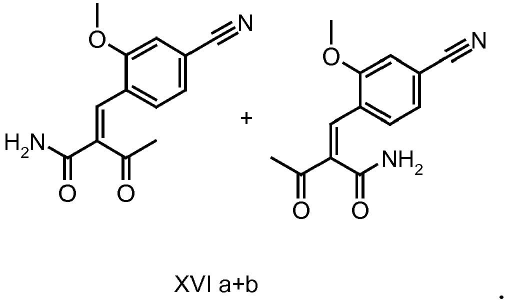

7. Compound of the formula 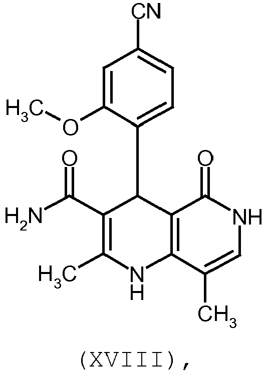 and the salts, solvates and solvates of the salts thereof.

8. Compound of the formula (XVI, a+b) as an E/Z mixture 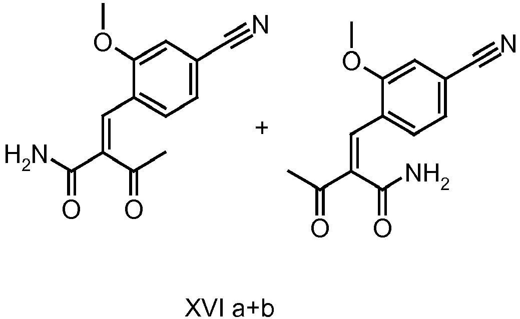 and the salts, solvates and solvates of the salts thereof.

9. Process for preparing the compound of the formula (XVIII) 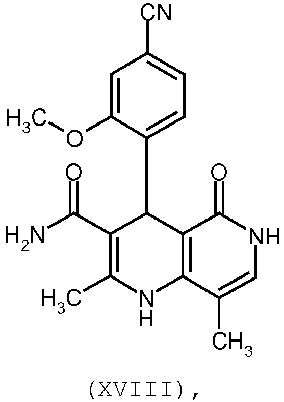 **characterized in that** the compounds of the formula (XVI a,b) 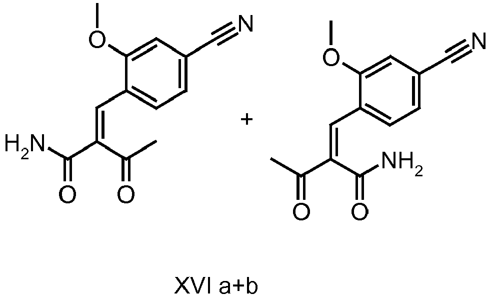 are reacted with the compound of the formula (IX)

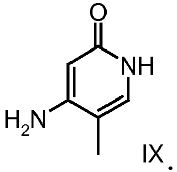

10. Process for preparing the compounds of the formula (XVI a,b) 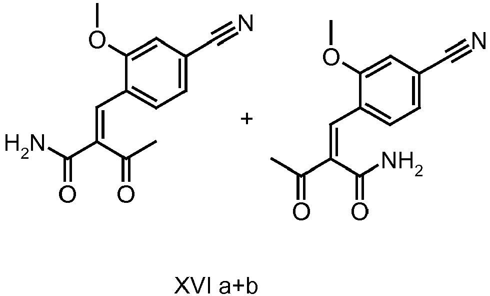 **characterized in that** the compound of the formula (VI) 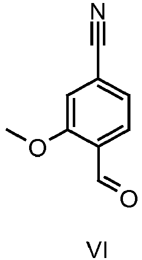 is reacted with the compound of the formula (XVII)

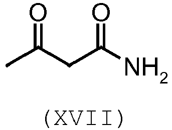

11. Process for preparing the compound (XIII) 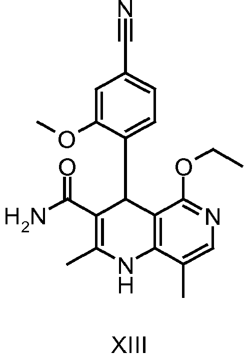 **characterized in that** the compound of the formula (XVIII) 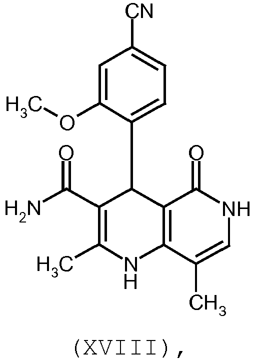 is reacted with the orthoester (XX) 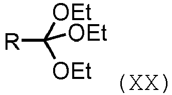 where R may be H or methyl.

12. Process for preparing the compound of the formula (XIII) 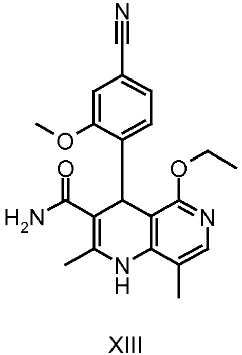 **characterized in that** the compound of the formula (XVIII) 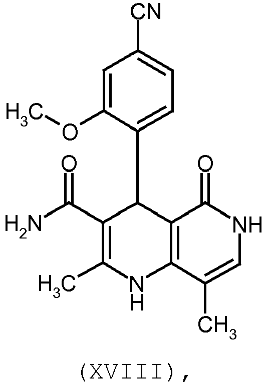 is reacted with the orthoester (XX) 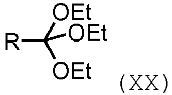 where R may be H or methyl, and
**characterized in that** the compound of the formula (XVIII) is prepared by reacting the compounds of the formula (XVI a,b) 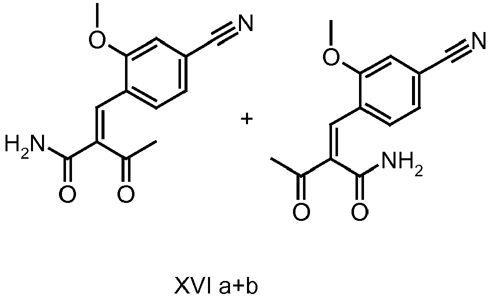 with the compound of the formula (IX)

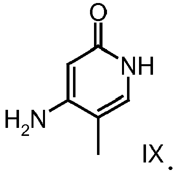

## Revendications

1. Procédé de fabrication du composé de formule (I) 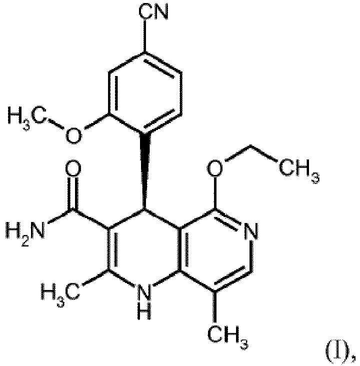 **caractérisé en ce que** le composé racémique de formule (XIII) est séparé en ses énantiomères, et le composé de formule (XIII) 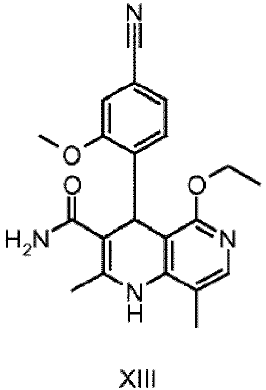 est fabriqué par mise en réaction du composé de formule (XVIII) 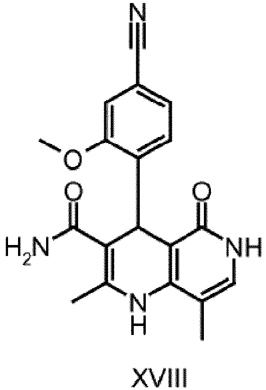 avec l'orthoester (XX) 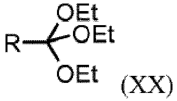 R pouvant représenter H ou méthyle, et le composé de formule (XVIII) 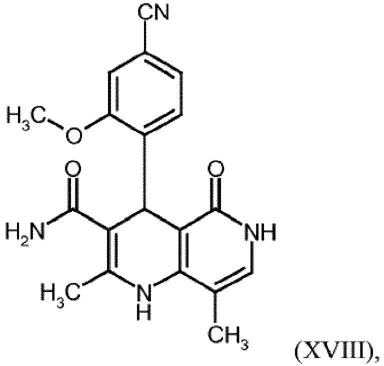 est fabriqué par mise en réaction des composés de formule (XVI a,b) 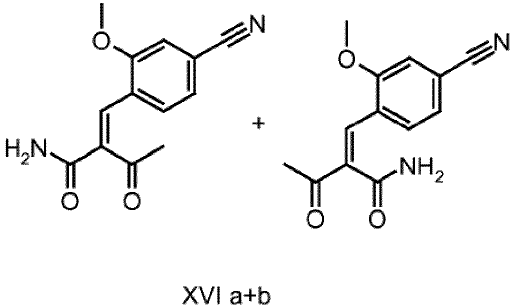 avec le composé de formule (IX) 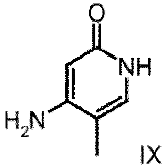 et
le composé de formule (XVI a,b) est fabriqué par mise en réaction du composé de formule (VI) 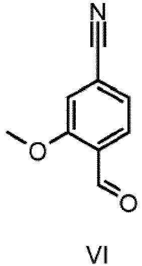 avec le composé de formule (XVII)

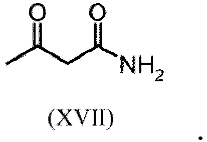

2. Procédé de fabrication du composé de formule (I) 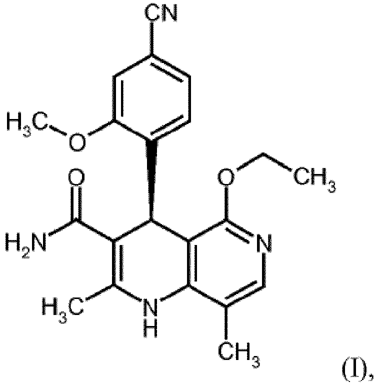 **caractérisé en ce que** le composé racémique de formule (XIII) est séparé en ses énantiomères, le composé de formule (XIII) 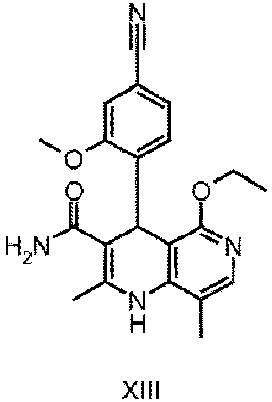 étant fabriqué par mise en réaction du composé de formule (XVIII) 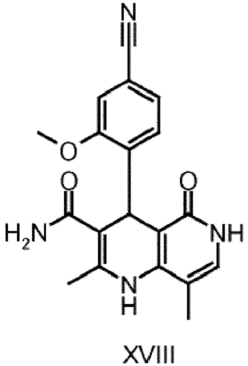 avec l'orthoester (XX) 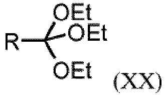 R pouvant représenter H ou méthyle, et
le composé de formule (XVIII) 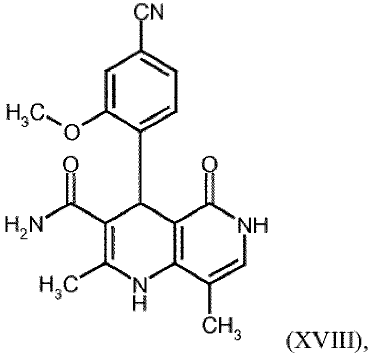 étant fabriqué par mise en réaction des composés de formule (XVI a,b) 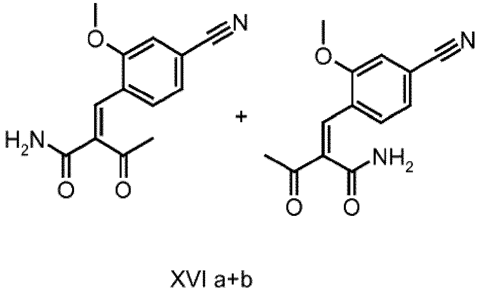 avec le composé de formule (IX)

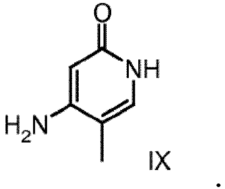

3. Procédé de fabrication du composé de formule (I) 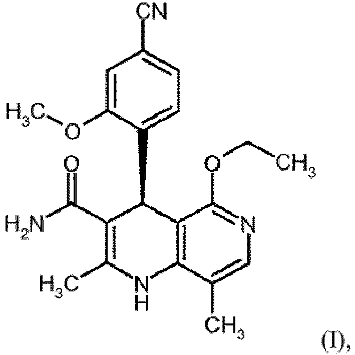 **caractérisé en ce que** le composé racémique de formule (XIII) est séparé en ses énantiomères, le composé de formule (XIII) 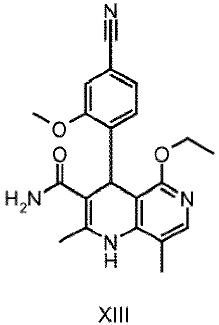 étant fabriqué par mise en réaction du composé de formule (XVIII) 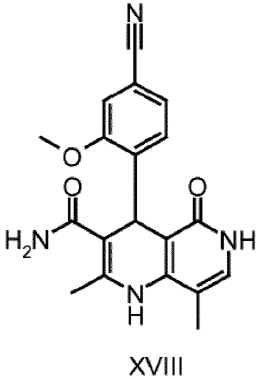 avec l'orthoester (XX) 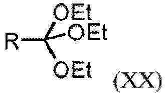 R pouvant représenter H ou méthyle.

4. Procédé de fabrication du composé de formule (I) 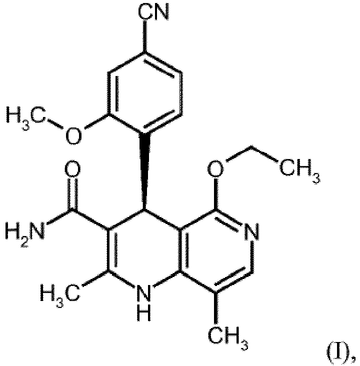 utilisant le composé de formule (XVIII)

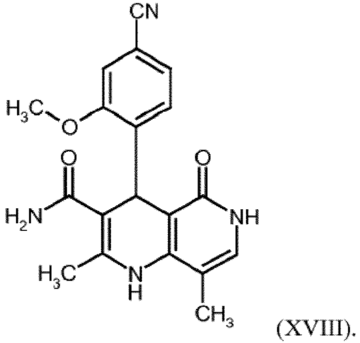

5. Procédé de fabrication du composé de formule (I) 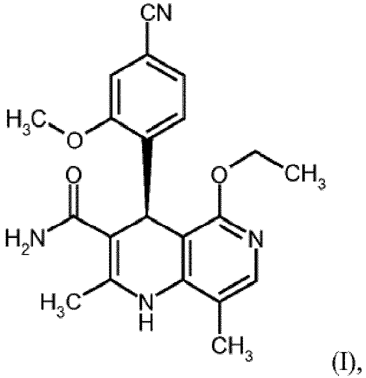 utilisant les composés de formule (XVI a,b)

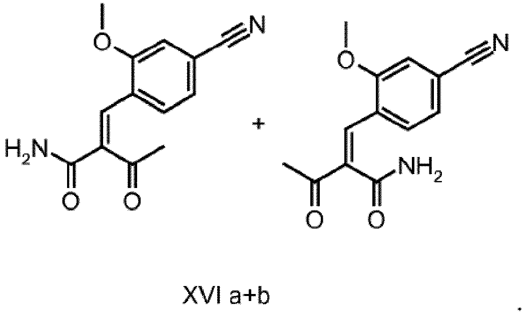

6. Procédé de fabrication du composé de formule (I) 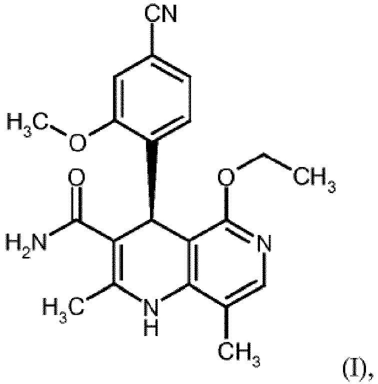 utilisant le composé de formule (XVIII) 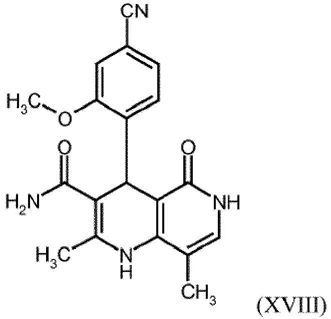 et les composés de formule (XVI a,b)

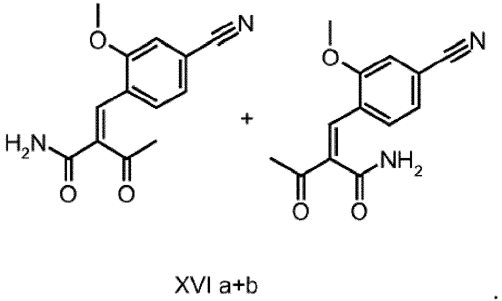

7. Composé de formule 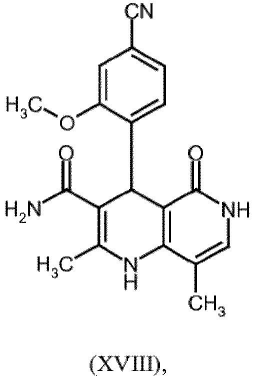 ainsi que ses sels, ses solvates et les solvates des sels.

8. Composé de formule (XVI a+b) sous la forme d'un mélange E/Z 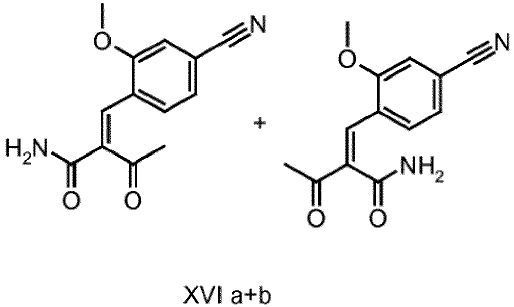 ainsi que ses sels, ses solvates et les solvates des sels.

9. Procédé de fabrication du composé de formule (XVIII) 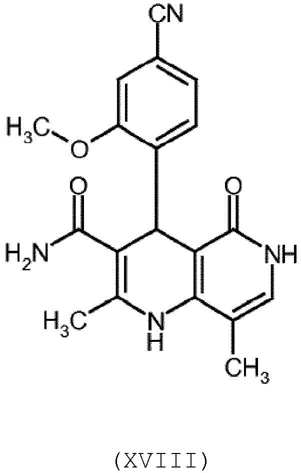 **caractérisé en ce que** les composés de formule (XVI a,b) 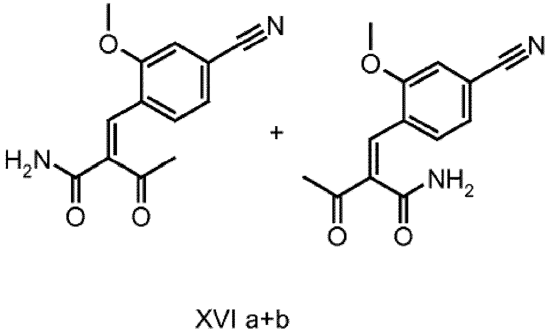 sont mis en réaction avec le composé de formule (IX)

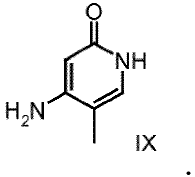

10. Procédé de fabrication des composés de formule (XVI a,b) 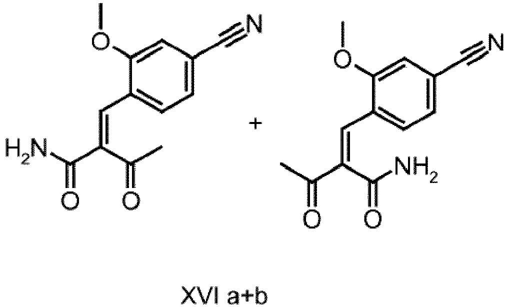 **caractérisé en ce que** le composé de formule (VI) 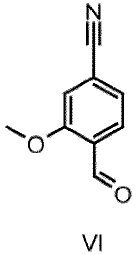 est mis en réaction avec le composé de formule (XVII)

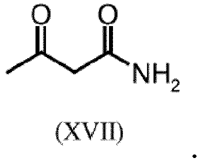

11. Procédé de fabrication du composé (XIII) 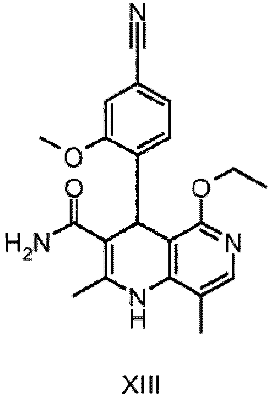 **caractérisé en ce que** le composé de formule (XVIII) 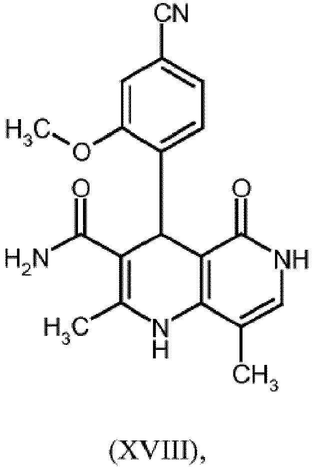 est mis en réaction avec l'orthoester (XX) 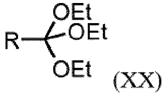 R pouvant représenter H ou méthyle.

12. Procédé de fabrication du composé de formule (XIII) 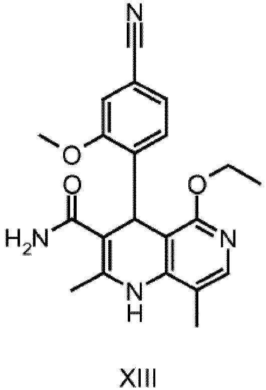 **caractérisé en ce que** le composé de formule (XVIII) 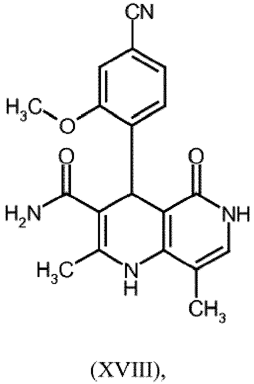 est mis en réaction avec l'orthoester (XX) 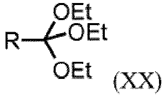 R pouvant représenter H ou méthyle, et
**caractérisé en ce que** le composé de formule (XVIII) est fabriqué par mise en réaction des composés de formule (XVI a,b) 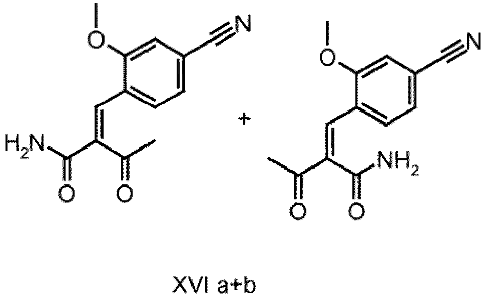 avec le composé de formule (IX)
